# EUROPÄISCHE PATENTSCHRIFT

(11) **EP 3 610 249 B1**
(45) Veröffentlichungstag und Bekanntmachung des Hinweises auf die Patenterteilung: **17.07.2024**
(21) Anmeldenummer: 18716600.4
(22) Anmeldetag: 06.04.2018
(51) Int. Cl.: G01N 23/223, A61B 6/00

(54) **VERFAHREN UND MESSVORRICHTUNG ZUR RÖNTGENFLUORESZENZ-MESSUNG**
METHOD AND MEASURING APPARATUS FOR AN X-RAY FLUORESCENCE MEASUREMENT
PROCÉDÉ ET DISPOSITIF DE MESURE PERMETTANT DE MESURER LA FLUORESCENCE DES RAYONS X

(30) Priorität: 11.04.2017 DE 102017003517
(43) Veröffentlichungstag der Anmeldung: 19.02.2020
(73) Patentinhaber: Axiom Insights GmbH, 22607 Hamburg (DE)
(72) Erfinder: GRÜNER, Florian, 22761 Hamburg (DE); HOESCHEN, Christoph, 39114 Magdeburg (DE); BLUMENDORF, Florian, 22761 Hamburg (DE)
(74) Vertreter: v. Bezold & Partner Patentanwälte - PartG mbB
(86) Internationale Anmeldenummer: PCT/EP2018/058859
(87) Internationale Veröffentlichungsnummer: WO 2018/189051

(56) Entgegenhaltungen:
- DE-A1- 102012 023 344
- US-A1- 2006 182 217
- US-A1- 2016 252 471
- BAZALOVA M ET AL: "Investigation of X-ray Fluorescence Computed Tomography (XFCT) and K-Edge Imaging", IEEE TRANSACTIONS ON MEDICAL IMAGING, IEEE SERVICE CENTER, PISCATAWAY, NJ, US, vol. 31, no. 8, August 2012 (2012-08-01), pages 1620 - 1627, XP011491136, ISSN: 0278-0062, DOI: 10.1109/TMI.2012.2201165
- QINGKAI HUO ET AL: "Sheet-beam geometry for in vivo fluorescent x-ray computed tomography: proof-of-concept experiment in molecular imaging", OPTICS LETTERS, OPTICAL SOCIETY OF AMERICA, US, vol. 33, no. 21, November 2008 (2008-11-01), pages 2494 - 2496, XP001519550, ISSN: 0146-9592, DOI: 10.1364/OL.33.002494

## Beschreibung

Die Erfindung betrifft ein Verfahren zur Röntgenfluoreszenz-Messung, insbesondere zur Röntgenfluoreszenz-Bildgebung, bei dem erfasst wird, ob fluoreszierende Targetpartikel in einem zu untersuchenden Objekt, insbesondere im Körper eines untersuchten Probanden, vorhanden sind, und vorhandene Targetpartikel im Objekt lokalisiert werden. Die Erfindung betrifft auch eine Röntgenfluoreszenz-Messvorrichtung, insbesondere eine Röntgenfluoreszenz-Bildgebungsvorrichtung, zur Ausführung eines derartigen Verfahrens. Anwendungen der Erfindung bestehen bei der Röntgenfluoreszenz-Bildgebung, insbesondere für medizinische Zwecke und dabei insbesondere für Objekte mit Größenskalen wie beim Menschen.

Ziele der medizinischen Bildgebung sind die Detektion, die Charakterisierung und das Monitoring von pathologischen Veränderungen des untersuchten Körpers und/oder die Pharmakokinetik, wobei die Verteilung von Medikamenten im Körper in-vivo gemessen wird. Um diese Methoden zu verbessern, werden Techniken der so genannten "molekularen Bildgebung" entwickelt, zum Beispiel um eine Tumor-Frühdiagnostik und bessere Charakterisierung des Tumorgewebes oder pharmakokinetische Untersuchungen zu erlauben. Die Charakterisierung erlaubt hierbei stratifizierte Behandlungsansätze, um so die Heilungschancen zu erhöhen. Die molekulare Bildgebung in der Medizin zielt insbesondere auf die Lokalisation von Biomarkern im Körper, wie z. B. Antikörper im Tumorgewebe, und erlaubt die in-vivo-Messung der Verteilung von Medikamenten im Körper, um zu untersuchen, welcher Anteil der applizierten Medikamentenmenge zu welcher Zeit sich an welchem Ort befindet. Dies könnte die Wirksamkeit von neuen Medikamenten bereits in der Erprobungsphase schneller testen, da Medikamente, die am Zielort nicht in ausreichender Menge vorhanden sind, keine klinische Wirkung entfalten können.

Bisher ist auf dem Gebiet der molekularen Bildgebung die Positron-Emissions-Tomographie (PET) etabliert, die jedoch als nuklearmedizinisches Verfahren für den Patienten entscheidende Nachteile hat. Zu diesen zählen die relativ hohe Dosis, die Exposition nicht betroffener Organe, die kurze Lebenszeit der Radiopharmaka, so dass Nachfolgeuntersuchungen eine neue Injektion erfordern, die geringe räumliche Auflösung bei 4 bis 6 mm, und die begrenzte zeitliche Auflösung, so dass relevante Prozesse bei Tumorversorgung oder Therapie nicht untersucht werden können. Insbesondere pharmakokinetische Untersuchungen über einen längeren Zeitraum sind damit effektiv unmöglich. Sollen Medikamente per Wirkstoffträger ("drug carrier") an den Zielort gebracht und dort freigesetzt werden, ist das "diagnostische Zeitfenster" von PET zu kurz.

Als Alternative zu PET wird schon seit vielen Jahren die Röntgenfluoreszenz-Bildgebung (RFB) diskutiert, mit der die Nachteile von PET vermieden werden können. Bei der RFB sind die Biomarker an z. B. Gold-Nanopartikel gebunden, die von einem scannenden Röntgenstrahl zur Emission von Röntgenfluoreszenz angeregt werden. Die Erfassung der Röntgenfluoreszenz erlaubt die Lokalisation der Biomarker. Da das RFB-Signal in eng begrenzten Energiebereichen im zu messendem Spektrum der vom Körper emittierten Röntgenstrahlung liegt, wird die Erfassung der Biomarker durch eine energieselektive Detektion der Röntgenfluoreszenz zwar begünstigt. Herkömmliche RFB-Verfahren zeichnen sich jedoch durch die im Folgenden dargestellten Nachteile aus, so dass sie bisher nur mit relativ geringer Sensitivität und für medizinische Anwendungen viel zu hohen Strahlungsdosen an Kleintiermodellen (Mäuse, Kleintierphantome) getestet werden konnten, während es für untersuchte Objekte der Größe eines Patienten bisher keine Untersuchungen gibt.

Ein Hauptnachteil des RFB-Verfahrens besteht darin, dass es im Gegensatz zu PET nicht untergrundfrei ist und die Amplitude des Untergrunds mit der Größe des untersuchten Objekts stark zunimmt. Herkömmliche RFB-Verfahren hätten daher bei großen Objekten eine viel zu geringe Sensitivität, so dass diese Techniken für den klinischen Einsatz bei Patienten nicht in Frage kämen. Aus Kleintiermodellen sind zwar die zu erwartenden Konzentrationen von z. B. Gold-Nanopartikeln bekannt (siehe z. B. N. Manohar et al. (2016) "Quantitative imaging of gold nanoparticle distribution in a tumor-bearing mouse using benchtop x-ray fluorescence computed tomography" in "Sci Rep" 6:22079), aber solche RFB-Verfahren könnten diese Gold-Nanopartikel-Mengen nicht im Menschen nachweisen, weil dann die Sensitivität nicht mehr ausreichend wäre.

Der RFB-Untergrund wächst mit der Größe des Objekts, da zugleich die Wahrscheinlichkeit zunimmt, dass die eingestrahlten Röntgenphotonen mehrfache Compton-Streuungen erfahren. Durch die Streuungen kann die Energie der Photonen so abgesenkt werden, dass die gestreuten Photonen im selben Energiebereich detektiert werden, wie die Fluoreszenz-Photonen. Da diese als Signal-Photonen im gemeinsamen Energiebereich nicht von den Untergrund-Photonen unterschieden werden können, ist die Sensitivität der Erfassung von Biomarkern beschränkt. Ohne eine Reduktion des Untergrunds können z. B. 1000 Signal-Photonen in einem Untergrund in dem fraglichen RFB-Energiebereich von rd. 1 Million Photonen untergehen, da das Signal statistisch nicht signifikant wäre. Bisher werden zur Reduktion des RFB-Untergrunds Kollimator-Geometrien vorgeschlagen, deren Funktion jedoch Vorab-Informationen erfordert, wo das Volumen mit dem angereicherten Kontrastmittel im Objekt liegt. Des Weiteren schränken herkömmliche Kollimatoren das Sichtfeld der Detektorelemente ein, wodurch Signal verloren geht, was nur durch eine höhere Bestrahlungsdosis kompensiert werden kann.

Schließlich ist die Röntgenfluoreszenz-Bildgebung bisher nur entweder an großen SynchrotronAnlagen oder mit herkömmlichen kompakten Röntgenröhren untersucht worden. Beide Verfahren eignen sich jedoch nicht für klinische Anwendungen, da Synchrotronanlagen zu groß sind und herkömmliche Röntgenröhren typischerweise Strahlung mit einer zu großen Divergenz und/oder zu geringen Intensität in Strahlen mit kleiner Divergenz emittieren und eine zu große Energiebandbreite aufweisen.

In DE 10 2012 023 344 A1 wird die Röntgenfluoreszenzanalyse einer Kontrastmittelverteilung in einem Objekt unter Verwendung einer kollimierten und im unteren Energiebereich gefilterten Röntgenquelle beschrieben. Dabei wird die durch Röntgenfluoreszenz erzeugte Röntgenstrahlung mit Kollimatorlamellen von mehrfach gestreuten Röntgenstrahlen getrennt und anschließend energieselektiv mit Monochromatorkristallschichten und einem Röntgendetektor gemessen. Durch Rotieren und Verschieben der Messvorrichtung um das Objekt wird eine computertomographische Bildgebung ermöglicht. Obwohl mit der Technik gemäß DE 10 2012 023 344 A1 eine medizinische Bildgebung an großen Objekten erzielt werden soll, ist die Anwendung in der Praxis aufgrund der folgenden Nachteile beschränkt. Erstens können mit den Monochromatorkristallschichten nur Fluoreszenzphotonen detektiert werden, die senkrecht zur Anregungsstrahlrichtung emittiert werden, da für alle anderen Richtungen die Bragg-Bedingung für Reflexion an den Monochromator-Kristallen nicht erfüllt ist. Das bedeutet insbesondere, dass ein Photon, welches z. B. am Anfang des Strahlvolumens im Objekt emittiert wird, jedoch nicht senkrecht zur Strahlrichtung, zwar die Lamellen passieren kann, aber an den Monochromatorkristallen nicht reflektiert wird, weil aus dieser Photon-Richtung die Bragg-Bedingung nicht erfüllt ist. Damit trägt dieses Photon, obwohl es ein Signal-Photon ist, nicht zum gemessen Signal bei, so dass die in DE 10 2012 023 344 A1 beschriebene Technik äußerst ineffizient ist. Zweitens ergibt sich durch die geringe Signalausbeute ein substantieller Nachteil durch ein geringes Signal-Rausch-Verhältnis.

Die Aufgabe der Erfindung ist es, ein verbessertes Verfahren zur Röntgenfluoreszenz-Messung, insbesondere zur Röntgenfluoreszenz-Bildgebung, und/oder eine verbesserte Röntgenfluoreszenz-Messvorrichtung bereitzustellen, mit denen Nachteile herkömmlicher Techniken vermieden werden. Die Erfindung soll insbesondere ermöglichen, die Röntgenfluoreszenz von Targetpartikeln in einem Objekt mit einer erhöhten Sensitivität zu detektieren, den Untergrund effizienter zu unterdrücken, die Bestrahlungsintensität zu senken und/oder die räumliche Auflösung zu verbessern. Die Erfindung soll des Weiteren insbesondere die bisher nicht mögliche Untersuchung von größeren Objekte zuzulassen und eine klinische Röntgenfluoreszenz-Bildgebung, insbesondere am Menschen, ermöglichen.

Diese Aufgaben werden durch ein Verfahren zur Röntgenfluoreszenz-Messung und eine Röntgenfluoreszenz-Messvorrichtung mit den Merkmalen der unabhängigen Ansprüche gelöst. Vorteilhafte Ausführungsformen und Anwendungen der Erfindung ergeben sich aus den abhängigen Ansprüchen.

Gemäß einem ersten allgemeinen Gesichtspunkt der Erfindung wird die oben genannte Aufgabe durch ein Verfahren zur Röntgenfluoreszenz-Messung gelöst, wobei das Vorhandensein von fluoreszierenden Targetpartikeln in einem zu untersuchenden Objekt erfasst und ggf. vorhandene Targetpartikel im Objekt zumindest in einer Projektionsebene (im Folgenden: erste Projektionsebene) lokalisiert werden.

Mit einer Quelleneinrichtung wird ein Röntgenstrahl erzeugt, der durch das Objekt in einer Röntgenstrahlrichtung parallel zu einer ersten Projektionsrichtung gerichtet wird. Der Röntgenstrahl ist vorzugsweise ein Nadelstrahl (Pencil-Strahl), der einen konstanten Durchmesser oder eine geringe Divergenz (z. B. ≤ 1mrad) aufweist. Der Durchmesser des Röntgenstrahls definiert die räumliche Auflösung des RFB-Verfahrens und beträgt daher vorzugsweise mindestens 0.1 mm, insbesondere mindestens 0.2 mm und/oder höchstens 1 mm, insbesondere höchstens 2 mm.

Das Objekt wird mit dem Röntgenstrahl an einer Vielzahl von Scanpositionen in der ersten Projektionsebene bestrahlt (abgescannt). Die Scanpositionen (Positionen, an denen eine Bestrahlung erfolgt) werden mit einer Scaneinrichtung eingestellt, mit der die Quelleneinrichtung und das Objekt relativ zueinander bewegt werden. Für jede Scanposition schneidet der Röntgenstrahl die erste Projektionsebene an anderen Koordinaten. Vorzugsweise ist der Röntgenstrahl senkrecht zur ersten Projektionsebene ausgerichtet. Die Scanpositionen sind in der ersten Projektionsebene z.B. als Matrix zeilen- und spaltenweise verteilt.

Zu jeder Scanposition wird die Röntgenstrahlung, die aus dem Objekt in eine Vielzahl von Raumrichtungen abgestrahlt wird, mit einer Detektorarrayeinrichtung detektiert, die mit der Quelleneinrichtung fest verbunden ist. Mit der Scaneinrichtung werden die Quellen- und Detektoreinrichtungen und das Objekt relativ zueinander bewegt. Vorzugsweise werden die Quellen- und Detektoreinrichtungen relativ zu einem ortsfest gehalterten Objekt bewegt.

Die Detektorarrayeinrichtung weist eine Anordnung von spektral selektiven Detektorelementen (auch als Pixel bezeichnet) auf, die in einer Vielzahl verschiedener Raumrichtungen um das Objekt verteilt sind. Die Detektorelemente sind entlang einer Arrayfläche angeordnet, die gekrümmt, abschnittsweise gekrümmt und/oder aus ebenen Teilflächen zusammengesetzt ist. Für jede Scanposition wird mit jedem spektral sensitiven Detektorelement ein Energiespektrum der aus dem Objekt austretenden detektierten Röntgen-Photonen gemessen und diese Einzelspektren werden zu einem Summen-Energiespektrum ausgewählter und/oder aller Detektorelemente zusammengesetzt. Die Messung von Energiespektren mit den Detektorelementen stellt einen wesentlichen Unterschied zu DE 10 2012 023 344 A1 vor, wo der Detektor nur Signal-Photonen messen kann, die eine bestimmte Energie aufweisen und senkrecht zur Röntgenstrahlrichtung emittiert werden. Des Weiteren hat die Bildung von Summen-Energiespektren (Summensignalen) gegenüber der Betrachtung der Spektren der einzelnen Detektorelemente insbesondere den Vorteil, dass bei den z.B. aus dem oben zitierten Kleintiermodell zu erwartenden, geringen Mengen an Targetpartikeln die Anzahl der Signal-Photonen in den einzelnen Detektorelementen so gering wäre, dass eine Bestimmung der Signifikanz erschwert oder sogar ausgeschlossen wäre.

Die Detektorelemente sind zur Detektion der Röntgenstrahlung in der Vielzahl von Raumrichtungen angeordnet. Das "Detektorelement" kann ein einzelnes, spektral auflösendes detektierendes Element, ein Verbund von mehreren (z. B. 4x4) detektierenden Elementen oder eine aus mehreren detektierenden Sub-Elementen zusammengesetzte, detektierende Komponente sein. Der Verbund von mehreren detektierenden Elementen kann insbesondere Vorteile für die Statistik der detektierten Röntgenphotonen haben. Die aus Sub-Elementen bestehende Komponente kann insbesondere Vorteile für die spektral auflösende Detektion bei der Verwendung einer laserbasierten Thomson-Quelle als Quelleneinrichtung haben, da hierbei die Photonen annähernd gleichzeitig detektiert werden und man durch die Sub-Elemente sicherstellt, dass pro Sub-Element nicht mehr als 1 Photon pro Detektor-Zeitfenster detektiert wird.

Die Detektorarrayeinrichtung weist des Weiteren eine Vielzahl von Blendenlamellen auf, die sich zwischen dem Objekt und den Detektorelementen in Radialrichtungen relativ zu dem Röntgenstrahl im Objekt erstrecken, die Detektorelemente von im Objekt einfach oder mehrfach, insbesondere außerhalb des Röntgenstrahls, gestreuter Röntgenstrahlung abschirmen und so angeordnet sind, dass die Detektorelemente Röntgenstrahlung von jeglichen Orten innerhalb des Volumens des Röntgenstrahls im Objekt detektieren können. Es kann insbesondere jedes einzelne Detektorelement Röntgenstrahlung von jeglichen Orten innerhalb des Volumens des Röntgenstrahls im Objekt detektieren. Die Blendenlamellen sind so angeordnet, dass vom gesamten Volumen des Röntgenstrahls im Objekt freie (nicht-blockierte) Sichtlinien zu den Detektorelementen verlaufen und vom übrigen, nicht unmittelbar bestrahlten Volumen im Objekt Sichtlinien zu den Detektorelementen durch die Blendenlamellen blockiert sind. Die Blendenlamellen können vorzugsweise ebene Blätter mit jeweils konstanter Dicke sein oder alternativ Dickengradienten aufweisen, die zum Strahl hin zulaufen. Aufgrund der endlichen Größe der Lamellen wird ein kleiner Anteil der Signal-Photonen absorbiert, was jedoch als Verfälschung der Röntgenfluoreszenz-Messung vernachlässigbar ist, da der Anteil der absorbierten Signal-Photonen vorzugsweise weniger als 10% beträgt.

Die Blendenlamellen liefern vorteilhafterweise einen ersten Beitrag zur Untergrund-Reduktion, indem Streustrahlung aus dem Volumen des Objekts außerhalb des aktuell an der Scanposition bestrahlten Volumens abgeschirmt wird.

Detektorsignale der Detektorelemente werden verarbeitet, um in der detektierten Röntgenstrahlung Röntgenfluoreszenz von Targetpartikeln zu erfassen und im Fall der Erfassung der Röntgenfluoreszenz die Targetpartikel im Objekt zu lokalisieren. Hierzu werden erfindungsgemäß für jede von einer Vielzahl von vorbestimmten Scanpositionen eine Teilmenge von Detektorelementen identifiziert (so genannte signifikante oder identifizierte Detektorelemente), deren Detektorsignale die Erfassung der Röntgenfluoreszenz der Targetpartikel mit einer im Vergleich zu den übrigen Detektorelementen erhöhten statistischen Signifikanz ermöglichen. Die Gruppe der signifikanten (oder: identifizierten) Detektorelemente wird z. B. jeweils für jede Scanpositionen oder nur für eine Auswahl von Scanpositionen (z. B. jede zweite Scanposition in der Matrix von Scanpositionen) gesucht. Dabei wird die Signifikanz eines Summensignal (Summen-Spektrum) der Detektorelemente (d. h. der identifizierten Teilmenge) mit der Signifikanz des Summensignal (Summen-Spektrum) von jeder anderen Teilmenge der Detektorelemente verglichen.

Wenn an mindestens einer Scanposition signifikante Detektorelemente gefunden werden, werden, basierend auf den Detektorsignalen der signifikanten Detektorelementen und ohne Berücksichtigung der Detektorsignalen der übrigen Detektorelemente, das Vorhandensein von Targetpartikeln und diese Scanposition, für welche die signifikanten Detektorelemente gefunden werden, als mindestens eine Target-Scanposition ermittelt. Die Target-Scanposition repräsentiert die Koordinaten in der ersten Projektionsebene, an denen die Targetpartikel lokalisiert sind. Andernfalls, wenn an keiner Scanposition signifikante Detektorelemente identifiziert werden, wird erfasst, dass im Objekt keine Targetpartikel nachweisbar sind.

Die Auswahl der signifikanten Detektorelemente liefert einen substantiellen, vorteilhafterweise den größten, Beitrag zur Untergrund-Reduktion. Dieser Gesichtspunkt der Erfindung basiert insbesondere auf der genauen Untersuchung des intrinsischen Untergrunds durch die Erfinder, der durch Vielfach-Compton-Streuung der eingestrahlten Photonen im Körper erzeugt wird. Entscheidend für die Reduktion des Untergrunds ist die Erkenntnis, dass der Untergrund richtungsabhängig, also anisotrop, ist- während das Fluoreszenz-Signal von den Targetpartikeln isotrop emittiert wird, also keine Richtung auszeichnet. Mit anderen Worten, die Erfinder haben festgestellt, dass die Untergrund-Röntgenstrahlung aufgrund der Vielfach-Compton-Streuung, insbesondere bei großen Objekten wie bei klinischen Anwendungen, eine ungleichmäßige räumliche Verteilung (Anisotropie) aufweist. An Scanpositionen, an denen der Röntgenstrahl auf Targetpartikel trifft und Röntgenfluoreszenz erzeugt wird, detektieren die Detektorelemente der Detektorarrayeinrichtung die Röntgenfluoreszenz in verschiedenen Raumrichtungen (relativ zur Röntgenstrahlrichtung) mit einem jeweils verschiedenen Untergrund. Durch die Suche nach den signifikanten Detektorelementen werden diejenigen Detektorelemente erfasst, deren Summen-Spektrum die Röntgenfluoreszenz mit einem relativ geringen Untergrundsignal im Vergleich zu den übrigen Detektorelementen detektiert wird. Im Ergebnis wird die quantitative Erkenntnis über die Anisotropie des intrinsischen Untergrunds in eine Reduktion des Untergrunds mit Hilfe richtungsabhängiger Detektion umgesetzt.

Die Erfinder haben insbesondere festgestellt, dass die Anisotropie der Photonen, die aufgrund der Vielfach-Compton-Streuung eine Energie im Signal-Energiebereich aufweisen, von der Energie der zur Anregung verwendeten Röntgen-Photonen abhängig ist. Die zur Anregung verwendeten Röntgen-Photonen haben vorzugsweise eine Energie, bei der die Anisotropie maximal ist. Vorteilhafterweise ermöglicht dies eine besonders wirksame Unterdrückung des Untergrundes. Die Energie, bei der die Anisotropie maximal ist, kann durch experimentelle Tests (z.B. an einem Phantom), bzw. numerische Simulationen, bestimmt werden. Besonders bevorzugt wird die Energie der Photonen des anregenden Röntgenstrahls in einem schmalen Energieintervall oberhalb der K-Kante des fluoreszierenden Elements in den Targetpartikeln gewählt, bei Gold-Targetpartikel in etwa bei 85 keV.

Mit Hilfe von Computersimulationen konnten die Erfinder zeigen, dass der Untergrund um einen Faktor von etwa 600 bis 1000 reduziert werden kann - mit einer entsprechend effektiven Steigerung der Signifikanz - und dies bei einem Testphantom mit Durchmesser von 30 cm und einer minimalen Dosis, welches den Anforderungen bei der Bildgebung am Menschen gerecht wird. Vorteilhafterweise können damit bei klinischen Anwendungen, z. B. in der Tumordiagnostik, geringe Mengen an Testpartikeln in Objekten wie z. B. Menschen nachgewiesen werden. Erreicht wird dies dadurch, dass sowohl Signal als auch Untergrund nur in bestimmten Richtungen gemessen werden, d. h. mit Hilfe des Summen-Spektrums der identifizierten Detektorelemente. Wie sich zeigt, bilden diese Richtungen nur z. B. ca. 30% bis 40% des gesamten Raumwinkels. Diese Einschränkung aber ist der Schlüssel zu der effektiven Steigerung der Signifikanz. Erst die erfindungsgemäß erzielte Steigerung der Signifikanz erlaubt die in der medizinischen Bildgebung am Menschen erforderliche Sensitivität.

Das erfindungsgemäße Verfahren unterscheidet sich von sämtlichen herkömmlichen RFB-Verfahren, bei denen meist nur in einer Richtung (meist 90 Grad und größer zur Einstrahlrichtung) gemessen wird und/oder Kollimatoren immer nur die Beobachtung eines Ausschnitts des Strahlvolumens erlauben. Eine quantitative Untersuchung über die einzelnen Beiträge sämtlicher Richtungen zur Gesamt-Signifikanz des Fluoreszenz-Signals für die Detektion entlang des gesamten Strahlvolumens war bisher nicht bekannt. Insbesondere gegenüber der Technik gemäß DE 10 2012 023 344 A1 wird eine erheblich verbesserte Ausbeute der Detektion von Fluoreszenz-Photonen aus allen Raumrichtungen und eine Detektion mit stark verringertem Untergrundsignal erzielt, um damit eine deutliche höhere Effizienz des Verfahrens mit Blick auf Dosis und Bestrahlungszeit zu erreichen.

Die Erfinder haben ferner festgestellt, dass eine isotrope Detektion in allen Raumrichtungen, bei der man ein maximales Fluoreszenz-Signal erwarten würde, zugleich eine maximale Untergrund-Detektion ergeben würde. Die daraus resultierende Signifikanz des Fluoreszenz-Signals wäre dann so klein, dass man, insbesondere bei Objekten der Größe menschlicher Probanden, nur derart große Mengen an z. B. Gold-Nanopartikeln messen könnte, die medizinisch irrelevant sind. Davon abweichend wird durch die Erfindung vorgeschlagen, durch die Auswahl der signifikanten Detektorelemente die Raumrichtungen, in denen Röntgenstrahlung detektiert wird, a priori auszuwählen. Numerische Analysen der Erfinder haben z. B. gezeigt, dass eine Detektion in ca. 40% aller möglichen Raumrichtungen eine erhebliche Untergrund-Reduktion liefert. Die erstmalig von den Erfindern ausgeführte quantitative Untersuchung der Anisotropie des Compton-Untergrunds bei großen Objekten lieferte insbesondere das der Erfindung zugrunde liegende Konzept, bei der Röntgenfluoreszenz-Messung nur ein Summen-Signal aus signifikanten (identifizierten) Detektorelementen zu messen.

Die Erfindung erlaubt eine derart starke Reduktion des intrinsischen Untergrunds, dass die Röntgen-Fluoreszenz-Bildgebung als Bildgebungsmodalität in der klinischen Praxis am Menschen anwendbar wird. Während die erreichbaren Sensitivitäten von herkömmlichen Techniken wie CT und MRT zu gering sind, um als Tumor-Frühdiagnostik zu funktionieren, erlaubt die erfindungsgemäße Röntgen-Fluoreszenz-Messung eine deutliche Steigerung der Sensitivität bei gleichzeitigem Umgehen der oben genannten Nachteile der PET-Bildgebung. Die Erfindung ermöglicht es, die Steigerung der Sensitivität zu quantifizieren. Damit kann das Potential der Röntgen-Fluoreszenz-Bildgebung im Vergleich zu den anderen Bildgebungs-Modalitäten quantitativ charakterisiert benannt werden.

Gemäß einem zweiten allgemeinen Gesichtspunkt der Erfindung wird die oben genannte Aufgabe durch eine Röntgenfluoreszenz-Messvorrichtung gelöst, die zur Lokalisierung von fluoreszierenden Targetpartikeln in einem zu untersuchenden Objekt eingerichtet ist und eine Halteeinrichtung zur Aufnahme des Objekts, eine Quelleneinrichtung zur Erzeugung eines Röntgenstrahls, der durch das Objekt in einer Röntgenstrahlrichtung parallel zu einer ersten Projektionsrichtung verläuft, eine Detektorarrayeinrichtung zur Detektion von Röntgenstrahlung, die aus dem Objekt in eine Vielzahl von Raumrichtungen abgestrahlt wird, eine Scaneinrichtung, mit der die Quellen- und Detektorarrayeinrichtungen und die Halteeinrichtung relativ zueinander beweglich sind, und eine Steuereinrichtung zur Aufnahme und Verarbeitung von Detektorsignalen der Detektorarrayeinrichtung umfasst. Vorzugsweise ist die Röntgenfluoreszenz-Messvorrichtung zur Ausführung des Verfahrens gemäß dem ersten allgemeinen Gesichtspunkt der Erfindung ausgelegt.

Die Quelleneinrichtung ist vorzugsweise eine kompakte laser-basierte Thomson-Quelle (Röntgenstrahlungsquelle, die Röntgenstrahlung basierend auf der Thomson-Streuung von Laserlicht an relativistischen Elektronen) erzeugt, wie sie z. B. von K. Khrennikov et al. ("Tunable All-Optical Quasimonochromatic Thomson X-Ray Source in the Nonlinear Regime") in "Phys. Rev. Lett.", Bd. 114, S. 195003 (2015) beschrieben wird, kann aber auch eine Synchrotronquelle oder allgemein eine Röntgenquelle, z. B. Röntgenröhre umfassen, die Röntgenstrahlung mit ausreichend geringer Divergenz und hoher Intensität insbesondere im Energiebereich oberhalb der K-Kante des Elements der Targetpartikel erzeugt. Die Detektorarrayeinrichtung ist mit der Quelleneinrichtung fest verbunden ist, und sie weist eine Vielzahl von spektral selektiven Detektorelementen und eine Vielzahl von Blendenlamellen auf, wie oben unter Bezug auf den ersten Gesichtspunkt der Erfindung beschrieben ist. Mit der Scaneinrichtung sind einerseits die Quellen- und Detektorarrayeinrichtungen und andererseits die Halteeinrichtung relativ zueinander so beweglich, dass der Röntgenstrahl das Objekt in der ersten Projektionsebene an einer Vielzahl von Scanpositionen abtasten kann.

Die Steuereinrichtung (auch als Recheneinrichtung, Auswertungseinrichtung oder Steuer- und Auswertungseinrichtung bezeichnet) ist dazu eingerichtet, für jede von einer Vielzahl von vorbestimmten Scanpositionen eine Teilmenge von signifikanten Detektorelementen zu identifizieren, deren Summen-Signal, vorzugsweise Summen-Spektrum, die Erfassung der Röntgenfluoreszenz der Targetpartikel mit einer im Vergleich zu den übrigen Detektorelementen erhöhten statistischen Signifikanz ermöglicht, und, wenn an mindestens einer Scanposition signifikante Detektorelemente gefunden werden, das Vorhandensein von Targetpartikeln zu erfassen und diese Scanposition als Target-Scanposition zu ermitteln, an der die Targetpartikel in der ersten Projektionsebene lokalisiert sind, oder, wenn an keiner Scanposition Detektorelemente identifiziert werden, zu erfassen, dass keine Targetpartikel nachweisbar sind. Die Steuereinrichtung ist z. B. ein Computer, auf dem ein Programm zur Ausführung der Verarbeitung der Detektorsignale läuft oder der als Spezialcomputer für die Ausführung der Verarbeitung der Detektorsignale konfiguriert ist. Die Steuereinrichtung ist mit den Komponenten der Röntgenfluoreszenz-Messvorrichtung gekoppelt, um beispielsweise die Quellen-, Scan- und Detektorarrayeinrichtungen anzusteuern oder von diesen Signale zu empfangen.

Gemäß einer bevorzugten Anwendung der Erfindung bei der Röntgenfluoreszenz-Bildgebung ist das untersuchte Objekt ein menschlicher oder tierischer Proband oder ein Körperteil von diesem. Dem Probanden wurden die Targetpartikel vorab zugeführt, z. B. durch orale oder anderweitige Verabreichung oder Injektion. Ein Vorbereitungsschritt mit einer Zufuhr von Targetpartikeln, insbesondere durch Injektion in den Körper des Probanden, ist nicht Teil der Erfindung. Alternativ können andere, nicht-biologische Objekte untersucht werden. Die Targetpartikel umfassen Partikel, insbesondere Nanopartikel, die zur Anregung von Röntgenfluoreszenz mit eingestrahlten Photonen, insbesondere mit einer Energie von mindestens 15 keV, geeignet sind und vorzugsweise eine Massenzahl im Bereich der Massenzahlen von lod bis Gold aufweisen. Diese Elemente haben den Vorteil, dass die K-Schalen-Fluoreszenz-Photonen eine ausreichende Transmission im Körper des Probanden haben, so dass die Targetpartikel in jeder Tiefe nachweisbar sind (im Gegensatz zur optischen Fluoreszenz). Des Weiteren sind die Targetpartikel mit einer Markersubstanz funktionalisiert. Die Markersubstanz (oder Biomarker) umfasst eine Substanz, die spezifisch an Teilen des untersuchten Objekts, z. B. an vorbestimmten Zellen oder Zellgruppen oder an vorbestimmtem Gewebe, wie z. B. Tumorzellen oder -gewebe, bindet. Die Targetpartikel können aber auch mit Medikamenten funktionalisiert sein, um damit in-vivo pharmakokinetische Untersuchungen zu erlauben.

Das Vorhandensein von fluoreszierenden Targetpartikeln in dem zu untersuchenden Objekt bedeutet, dass vorab eingebrachte, z. B. oral oder anderweitig eingenommene oder injizierte, Targetpartikel in mindestens einem Teilbereich des untersuchten Objekts durch die spezifische Bindung angereichert wurden. Die Erfassung des Vorhandenseins von Targetpartikeln umfasst die Erfassung einer Anreicherung (Konzentration) der Targetpartikel in dem mindestens einen Teilbereich, z. B. an Gewebe oder Zellgruppen. Die Lokalisierung der Targetpartikel umfasst die Erfassung des Ortes der Anreicherung zumindest in Bezug auf die erste Projektionsebene, vorzugsweise jedoch in Bezug auf alle drei Raumdimensionen. Die Erfindung hat insbesondere die folgenden Vorteile für medizinische Anwendungen. Der intrinsische Untergrund bei der medizinischen Röntgen-Fluoreszenz-Bildgebung kann so stark reduziert werden, dass bei vertretbarer Strahlendosis minimale Mengen an funktionalisierten Gold-Nanopartikel im Körper nachgewiesen werden können, um so z. B. eine Tumor-Frühdiagnostik zu ermöglichen.

Gemäß einer bevorzugten Ausführungsform der Erfindung wird für jede der Vielzahl von vorbestimmten Scanpositionen die Teilmenge der signifikanten Detektorelemente so gesucht, dass die Summen-Signale der signifikanten Detektorelemente die Erfassung der Röntgenfluoreszenz der Targetpartikel mit einer maximalen statistischen Signifikanz ermöglichen. Die Steuereinrichtung ist konfiguriert, die Detektorelemente auszuwählen, aus deren Summen-Signal die gesuchte Röntgenfluoreszenz der Targetpartikel mit maximaler statistischer Signifikanz erfasst werden kann. Vorteilhafterweise wird damit die maximale Sensitivität der Röntgenfluoreszenz-Messung erzielt. Maximale Sensitivität bedeutet dabei insbesondere, dass eine jede andere Teilmenge von Detektorelementen ein Summen-Signal mit geringerer Signifikanz des Signals aufweist.

Vorteilhafterweise sind verschiedene Verfahren verfügbar, die Teilmenge der signifikanten Detektorelemente zu identifizieren. Gemäß einer ersten bevorzugten Variante ist ein einstufiges Verfahren vorgesehen, bei dem die Detektorsignale der Detektorelemente an der betrachteten Scanposition, vorzugsweise mit der Steuereinrichtung, schrittweise einer Analyse des Auftretens der gesuchten Röntgenfluoreszenz der Targetpartikel unterzogen werden, wobei die statistische Signifikanz der Röntgenfluoreszenz im Summensignal der Detektorsignale des betrachteten und der verbleibenden Detektorelemente untersucht wird. Es werden die Detektorelemente verworfen, deren Detektorsignale keine Erhöhung der statistischen Signifikanz des Summensignals der verbleibenden Detektorelemente liefern. Alle nicht verworfenen Detektorelemente bilden die Gruppe der signifikanten Detektorelemente. Das einstufige Verfahren hat insbesondere den Vorteil, dass die signifikanten Detektorelemente mit hoher Prozessgeschwindigkeit ermittelt werden können.

Gemäß einer zweiten bevorzugten Variante ist ein zweistufiges Verfahren vorgesehen, bei dem in einem ersten Auswahlschritt Detektorelemente verworfen werden, die überwiegend Untergrund-Röntgenstreustrahlung detektieren, und in einem zweiten Auswahlschritt weitere Detektorelemente verworfen werden, deren Detektorsignale keine Erhöhung der statistischen Signifikanz des Summensignals der verbleibenden Detektorelemente liefern. Die Erfassung der Detektorelemente, die überwiegend Untergrund-Röntgenstreustrahlung detektieren, beim ersten Auswahlschritt basiert auf der spektral selektiven Detektion mit den Detektorelementen. (Untergrund-) Energiebereiche, in denen ausschließlich Untergrund-Photonen auftreten können, werden als Referenz zur Beurteilung der (Fluoreszenz-)Energiebereiche verwendet, in denen sowohl Untergrund- als auch Fluoreszenz-Photonen auftreten können. Liefert das Detektorsignal eines betrachteten Detektorelements im Untergrund-Energiebereich eine Amplitude, die sich statistisch nicht signifikant von der Signalamplitude in einem reinen Untergrund-Energiebereich unterscheidet, insbesondere deutlich größer ist als die zu erwartende Signal-Amplitude (im Fluoreszenz-Bereich), so wird das Detektorelement als überwiegend Untergrund-Röntgenstreustrahlung detektierend erfasst und somit verworfen. Das vorzugsweise mit der Steuereinrichtung ausgeführte zweistufige Verfahren hat insbesondere den Vorteil, dass die signifikanten Detektorelemente mit Genauigkeit und Reproduzierbarkeit ermittelt werden können.

Vorteilhafterweise können insbesondere die genannten Varianten mit einer Vorauswahl kombiniert werden, bei der für jede der Vielzahl von vorbestimmten Scanpositionen eine initiale Teilmenge von Detektorelementen vorgewählt wird, die vorab aufgrund von a priori Information (Vorab-Information) über das untersuchte Objekt ermittelt wird, wobei die Teilmenge der zu identifizierenden, signifikanten Detektorelemente innerhalb der vorgewählten initialen Teilmenge gesucht wird. Die a priori Information kann z. B. auf numerischen Simulationen, experimentellen Simulationen, vorhandenen Bildern des Objekts, z. B. Patientenaufnahmen aus CT oder MRT-Untersuchungen, und/oder einer vorbekannten Lokalisierung der Targetpartikel, z. B. eine Krankheitslokalisation (z.B. ein Lebertumor) oder als Zielvolumen für pharmakokinetische Untersuchungen basieren. Vorteilhafterweise wird die Auswahl der signifikanten Detektorelemente bei Betrachtung der initialen Teilmenge vereinfacht und die Prozessgeschwindigkeit erhöht.

Gemäß einer weiteren vorteilhaften Ausführungsform der Erfindung erfolgt die Röntgenfluoreszenz-Messung zweistufig mit einer Vorbereitungsmessung und einer Hauptmessung, bei denen das erfindungsgemäße Verfahren unter Verwendung eines Röntgenstrahls jeweils mit einem ersten, größeren, und einem zweiten, z. B. geringeren Durchmesser mit verschiedener örtlicher Auflösung ausgeführt werden. Der erste Strahl muss allerdings nicht zwingend größer sein. Er kann auch genauso groß sein wie der zweite Strahl, wobei jedoch weniger Dosis appliziert wird und anschließend benachbarte Pixel aufaddiert werden, um ausreichend Daten für die statistische Bewertung zu erhalten. Diese Variante hat den Vorteil, dass die Daten des Vorabscans für die zweite Messung weiterverwendet werden können. Bei der Vorbereitungsmessung wird mit dem ersten Röntgenstrahl mit dem ersten Durchmesser bei Erfassung des Vorhandenseins der Targetpartikel eine Vorbereitungs-Target-Scanposition ermittelt, die einen Target-Scanbereich in der ersten Projektionsebene darstellt. Bei der Hauptmessung mit dem zweiten Röntgenstrahl mit dem zweiten Durchmesser wird die gesuchte Target-Scanposition innerhalb des Target-Scanbereichs ermittelt. Vorteilhafterweise kann die Vorbereitungsmessung verwendet werden, um mit großer Geschwindigkeit festzustellen, ob im Objekt Targetpartikel erfassbar und in welchem Target-Scanbereich sie ggf. lokalisiert sind. Wenn keine Röntgenfluoreszenz von Targetpartikeln erfasst wird, kann die Hauptmessung unterbleiben. Wenn Röntgenfluoreszenz von Targetpartikeln erfasst wird, liefert die Hauptmessung die genaue Lokalisierung der Targetpartikel, wobei durch die Beschränkung ausschließlich auf den Target-Scanbereich ("Hineinzoomen" in das Targetgebiet) der Zeitaufwand der Hauptmessung vermindert werden kann.

Die Detektorarrayeinrichtung kann das Objekt mit Ausnahme von Raumwinkelbereichen zur Einstrahlung des Röntgenstrahls durch ein Einstrahlungsfenster und ggf. zur Einführung des Objekts in die Detektorarrayeinrichtung durch ein Einführungsfenster vollständig umgeben (4π-Detektor). Das Einstrahlungsfenster hat eine geringe Lateraldimension, die an den Durchmesser des Röntgenstrahls angepasst ist. Das Einführungsfenster hat eine Lateraldimension, die von der Form und Größe des Objekts und ggf. Teilen der Halteeinrichtung abhängt. Vorteilhafterweise werden damit nahezu alle Röntgenphotonen aus dem Objekt erfasst.

Gemäß einer alternativen Ausführungsform der Erfindung umfasst die Detektorarrayeinrichtung eine Anordnung der Detektorelemente auf einer Fläche, die nur, insbesondere näherungsweise, einen Halbraum in Vorwärtsrichtung des Röntgenstrahls abdeckt. Die Erfinder haben festgestellt, dass die signifikanten Detektorelemente überwiegend in dem Halbraum in Vorwärtsrichtung gefunden werden. Vorteilhafterweise ermöglicht diese Ausführungsform der Erfindung einen vereinfachten Aufbau der Detektorarrayeinrichtung und einen vereinfachten Zugriff auf das untersuchte Objekt.

Gemäß einer weiteren bevorzugten Ausführungsform der Erfindung kann die Detektorarrayeinrichtung eine Anordnung der Detektorelemente entlang einer Kugelfläche und/oder einer Zylinderfläche umfassen. In diesem Fall wird die radiale Anordnung der Blendenlamellen vereinfacht.

Die Lokalisierung der Targetpartikel in der ersten Projektionsebene liefert allgemein bereits eine auswertbare Bildinformation über das untersuchte Objekt und die Lage der Targetpartikel in diesem. Für eine vervollständigte Bildgebung ist gemäß einer besonders bevorzugten Ausführungsform der Erfindung nach der Lokalisierung der Targetpartikel in der ersten Projektionsebene eine Verschwenkung der Quellen- und Detektorarrayeinrichtungen relativ zum Objekt derart vorgesehen, dass im verschwenkten Zustand der Röntgenstrahl parallel zu einer zweiten Projektionsrichtung durch das Objekt verläuft, die von der ersten Projektionsrichtung abweicht. Zur Ausführung der Verschwenkung ist die Röntgenfluoreszenz-Messvorrichtung mit einer Schwenkeinrichtung ausgestattet, mit der die Quellen- und Detektorarrayeinrichtungen, vorzugsweise gemeinsam mit der Scaneinrichtung, so verschwenkbar relativ zur Halteeinrichtung des Objekts sind, dass der Röntgenstrahl parallel zu der zweiten Projektionsrichtung verläuft.

Im verschwenkten Zustand erfolgen eine weitere Bestrahlung des Objekts mit dem Röntgenstrahl an einer Vielzahl von Scanpositionen, die in diesem Fall auf eine Abtastlinie in einer zweiten Projektionsebene, die von der ersten Projektionsebene abweicht, beschränkt wird, wobei die Abtastlinie die vorher ermittelte Target-Scanposition enthält, und eine Erfassung der Position der Targetpartikel entlang der Abtastlinie, vorzugsweise mit der Steuereinrichtung. Mit der Ortsinformation über die Scanposition in der ersten Projektionsebene und die Scanposition auf der Abtastlinie in der zweiten Projektionsebene ist die Position der Targetpartikel im Objekt vollständig charakterisiert.

Die zweite Projektionsrichtung ist allgemein nicht parallel zur ersten Strahlrichtung, d. h. sie muss lediglich erlauben, dass die Scan-Richtungen sich im Raum schneiden können, wobei dieser Schnittpunkt dann die Lokalisation der Targetpartikel markiert, wenn beide Scan-Richtungen jeweils ein Signal anzeigen. Gemäß einer bevorzugten Variante der Erfindung ist jedoch vorgesehen, dass die Verschwenkung der Quellen- und Detektorarrayeinrichtungen so erfolgt, dass die zweite Projektionsrichtung senkrecht zu der ersten Projektionsrichtung und die zweite Projektionsebene senkrecht zu der ersten Projektionsebene ausgerichtet sind.

Gemäß einer weiteren bevorzugten Ausführungsform der Erfindung kann eine Aufnahme von mindestens einem Absorptionsprojektionsbild des Objekts vorgesehen sein. Vorteilhafterweise erfüllen die Detektorelemente in diesem Fall eine Doppelfunktion zur Erfassung der Röntgenfluoreszenz und zur Erfassung eines herkömmlichen Röntgen-Absorptionsbildes, z. B. zur Ermittlung von anatomischen Informationen über einen untersuchten Probanden. Vorzugsweise wird das Absorptionsprojektionsbild mit der Target-Scanposition in der ersten Projektionsebene oder der vollständigen Charakterisierung der Targetposition im Raum kombiniert, um ein Objektbild z. B. für eine nachfolgende Diagnose zu erhalten.

Weitere Einzelheiten der Erfindung werden im Folgenden unter Bezug auf die beigefügten Zeichnungen beschrieben. Es zeigen:
- Figuren 1 bis 6:: schematische Ansichten bevorzugten Ausführungsformen der erfindungsgemäßen Röntgenfluoreszenz-Messvorrichtung mit einer Anordnung von Detektorelementen auf einer Kugelfläche;
- Figuren 7 bis 9:: schematische Ansichten bevorzugten Ausführungsformen der erfindungsgemäßen Röntgenfluoreszenz-Messvorrichtung mit einer Anordnung von Detektorelementen auf einer Zylinderfläche;
- Figuren 10 und 11:: Flussdiagramme zur Illustration von Merkmalen bevorzugter Ausführungsformen des erfindungsgemäßen Verfahrens;
- Figur 12:: eine graphische Illustration der Auswahl signifikanter Detektorelemente;
- Figur 13:: eine graphische Illustration des spektralen Summensignals an einer TargetScanposition, und
- Figur 14:: eine graphische Illustration der erfindungsgemäß erzielten Untergrundunterdrückung anhand der messbaren Röntgenspektren.

Ausführungsformen der Erfindung werden im Folgenden unter Bezug auf die Merkmale der Röntgenfluoreszenz-Messvorrichtung und des Verfahrens insbesondere zur Erfassung des Vorhandenseins von fluoreszierenden Targetpartikeln in einem zu untersuchenden Objekt und, bei Erfassung von Targetpartikel-Fluoreszenz, zur Lokalisierung der Targetpartikel beschrieben. Einzelheiten der Röntgenfluoreszenz-Messvorrichtung, wie z. B. Einzelheiten der Quelleneinrichtung, der Scaneinrichtung, der Detektorelemente oder der Schwenkeinrichtung können realisiert werden, wie sie an sich von entsprechenden mechanischen, elektrischen oder röntgenoptischen Komponenten von herkömmlichen Techniken bekannt sind, so dass sie hier im Detail nicht beschrieben werden. Für Anwendungen bei der medizinischen Bildgebung kann die Röntgenfluoreszenz-Messvorrichtung mit weiteren Komponenten ausgestattet sein, wie es an sich von herkömmlichen Techniken bekannt ist, wie z. B. ein Antrieb zur Betätigung einer Halteeinrichtung, Bedienvorrichtungen, eine Anzeigevorrichtung u. dgl..

Es wird beispielhaft auf Ausführungsformen Bezug genommen, bei denen für die Einstellung der Scanpositionen die Quellen- und Detektorarrayeinrichtungen relativ zum ortsfest positionierten Objekt bewegt werden. Die Erfindung ist entsprechend, z. B. bei ortsfesten Quellen, wie herkömmlichen Synchrotronquellen, so anwendbar, dass das Objekt relativ zu ortsfest positionierten Quellen- und Detektorarrayeinrichtungen bewegt wird.

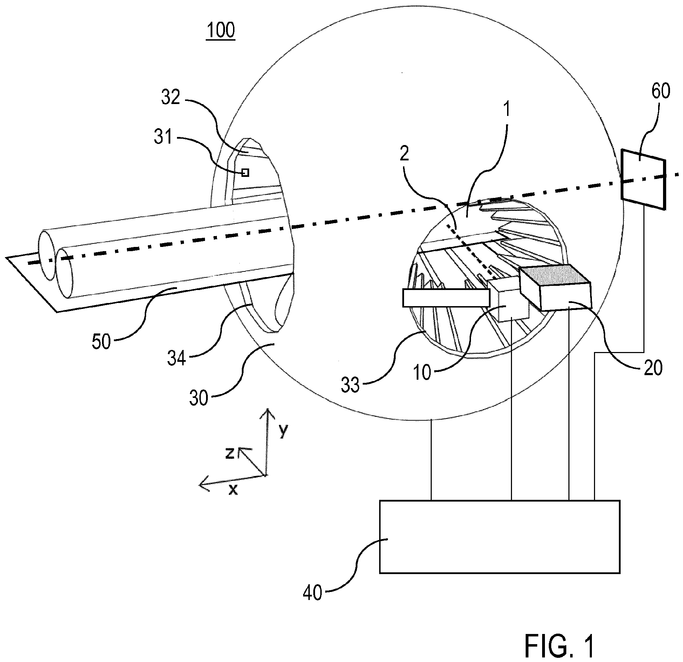

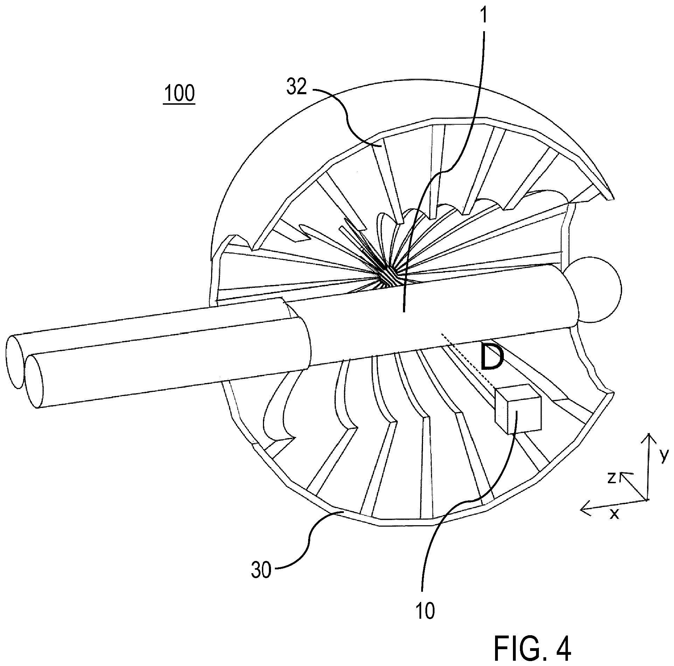

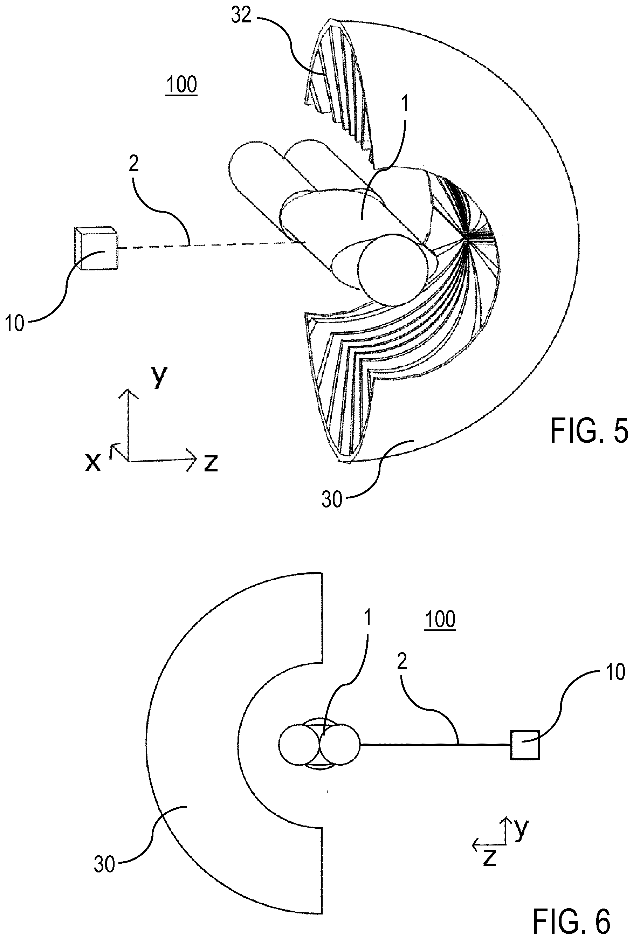

Die Figuren 1 bis 6 zeigen schematisch verschiedene Perspektiven der Röntgenfluoreszenz-Messvorrichtung 100 mit einer Anordnung von Detektorelementen 31 (ein Detektorelement ist beispielhaft gezeigt) auf einer Kugelfläche, wobei die Anordnung der Detektorelemente 31 das Objekt nahezu vollständig (Figuren 1 bis 3) oder nur in einem Halbraum in Vorwärtsrichtung des Röntgenstrahls (Figuren 4 bis 6) umgibt.

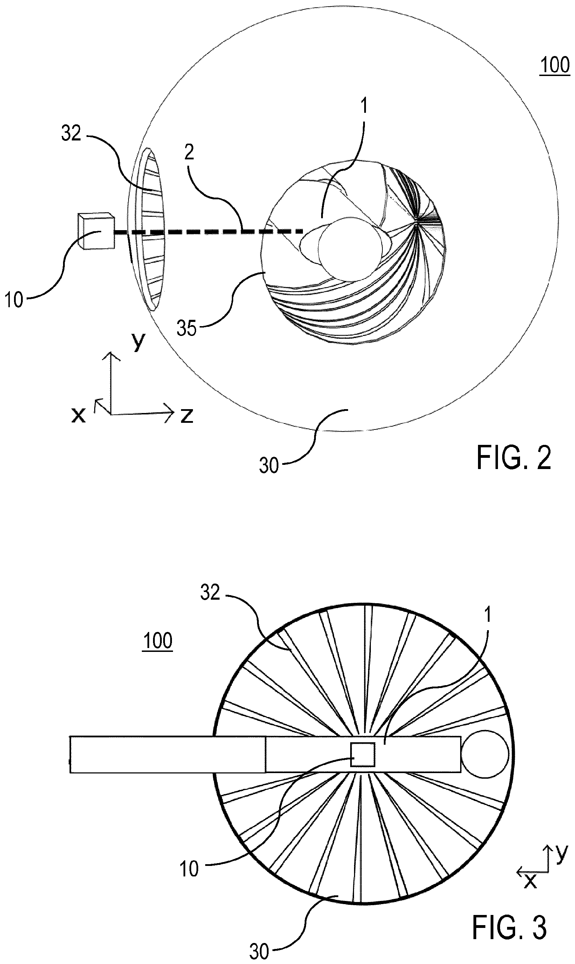

Die Röntgenfluoreszenz-Messvorrichtung 100 umfasst gemäß Figur 1 jeweils eine Halteeinrichtung 50, z. B. eine Liege, zur Aufnahme des Objekts 1, z. B. ein schematisch gezeigter Patient, eine Quelleneinrichtung 10 zur Erzeugung eines Röntgenstrahls 2 in einer ersten Projektionsrichtung (z-Richtung), eine Scaneinrichtung 20, eine Detektorarrayeinrichtung 30, die mit der Quelleneinrichtung 10 fest verbunden ist (nur in Figur 1 schematisch gezeigt), eine Steuereinrichtung 40 zur Aufnahme und Verarbeitung von Detektorsignalen der Detektorarrayeinrichtung 30 und eine optional vorgesehene Schwenkeinrichtung 60. Mit der Scaneinrichtung 20, die vorzugsweise einen mechanischen Antrieb mit einem Stellmotor enthält, können die Quellen- und Detektorarrayeinrichtungen 10, 30 so zeilen- und spaltenweise parallel zur ersten Projektionsebene (x-y-Ebene) bewegt werden, dass der Röntgenstrahl 2 senkrecht zur ersten Projektionsebene durch das Objekt 1 gescannt wird. Mit der Schwenkeinrichtung 60 können die Quellen- und Detektorarrayeinrichtungen 10, 30 gemeinsam mit der Scaneinrichtung 20 so gedreht werden, dass der Röntgenstrahl in eine zweite Projektionsrichtung (z. B. negative y-Richtung) gerichtet ist. In den übrigen Figuren 2 bis 6 sind die Halteeinrichtung, die Scaneinrichtung, die Steuereinrichtung und die Schwenkeinrichtung nicht gezeigt, aber wie in Figur 1 dargestellt vorgesehen.

Die Quelleneinrichtung 10 ist z. B. eine Quelle von Typ laser-basierte Thomson-Quelle. Die Detektorarrayeinrichtung 30 hat die Gestalt einer Hohlkugel mit einem Innendurchmesser von z. B. 120 cm, auf deren Innenoberfläche die Detektorelemente 31 und zwischen diesen in die Hohlkugel ragend Blendenlamellen 32 angeordnet sind. Die Hohlkugel ist eine Vollkugel (Figuren 1 bis 3) oder eine Halbkugel (Figuren 4 bis 6). Die Detektorelemente 31 und die Blendenlamellen 32 sind schematisch gezeigt. In der Praxis wird die Zahl und Größe der Detektorelemente und die Zahl, Größe und Ausrichtung der Blendenlamellen 32 in Abhängigkeit von der konkreten Messaufgabe gewählt. Die Detektorelemente 31 sind für die Detektion von Spektren der im Objekt 1 emittierten Röntgenstrahlung eingerichtet, sie umfassen z. B. Detektorelemente vom Typ CdTe (Hersteller z. B. Amptek). Die Blendenlamellen 32 sind Bleche mit einer zur Ausdehnung des im Objekt bestrahlten Volumens, d. h. zum Röntgenstrahl 2 hin sich verringernden Dicke (siehe schematische Schnittdarstellung in Figur 3) oder planare Blenden. Die Blendenlamellen 32 bestehen zum Beispiel aus Molybdän. Es sind z. B. bis zu 3600 Blendenlamellen 32 vorgesehen. Die Detektorarrayeinrichtung 30 hat ferner zwei Öffnungen, umfassend ein Einstrahlungsfenster 33 und ein Einführungsfenster 34 für die Halteeinrichtung 50 mit dem Objekt 1. Zusätzlich kann ein weiteres Zugriffsfenster 35 vorgesehen sein (siehe Figur 2).

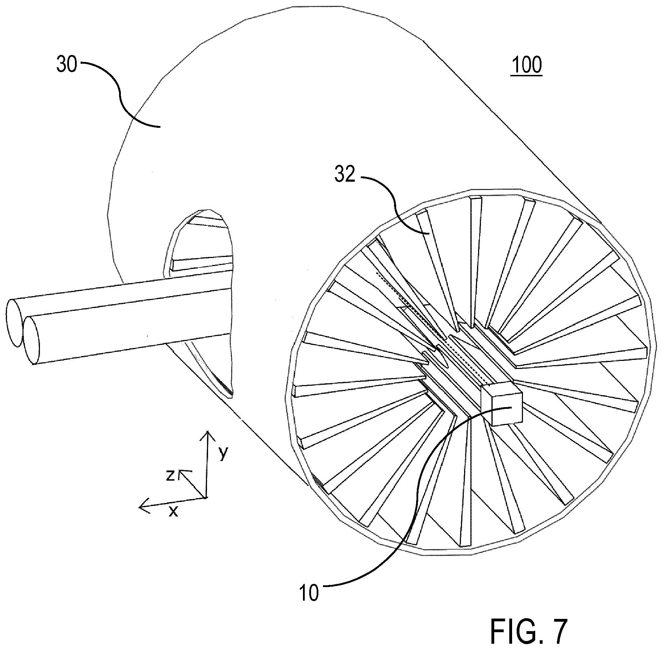

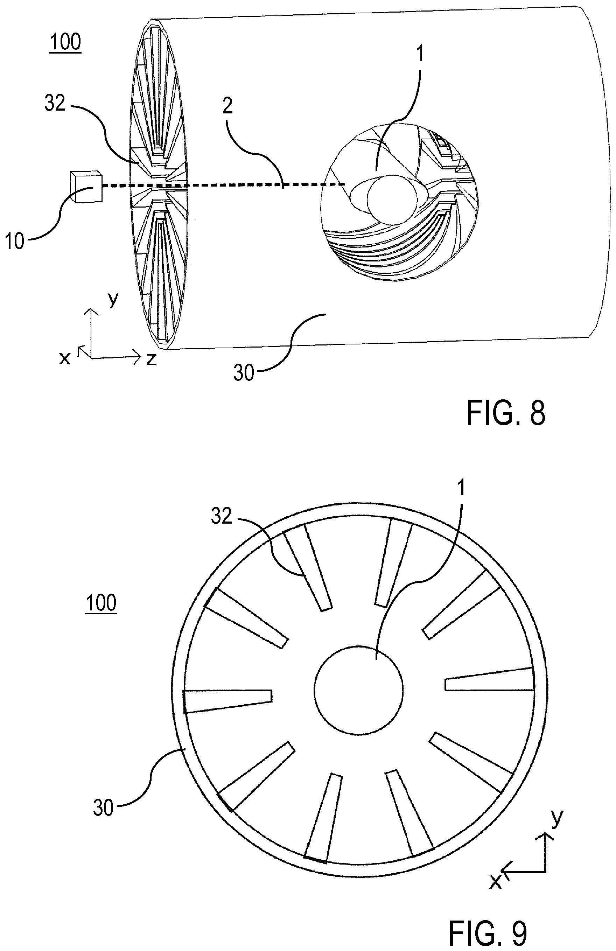

Die Figuren 7 bis 9 zeigen schematisch verschiedene Perspektiven der Röntgenfluoreszenz-Messvorrichtung 100 mit einer Anordnung von Detektorelementen 31 auf einer Zylinderfläche, wobei Detektorelemente auf ebenen Stirnflächen des Zylinders nicht gezeigt sind. Das Objekt wird nahezu vollständig von den Detektorelementen eingeschlossen. Alternativ kann eine Detektion in einem Halbraum in Vorwärtsrichtung des Röntgenstrahls vorgesehen sein (nicht dargestellt). In den Figuren 7 bis 9 sind die Halteeinrichtung, die Scaneinrichtung, die Steuereinrichtung und die Schwenkeinrichtung nicht gezeigt, aber wie in Figur 1 dargestellt vorgesehen.

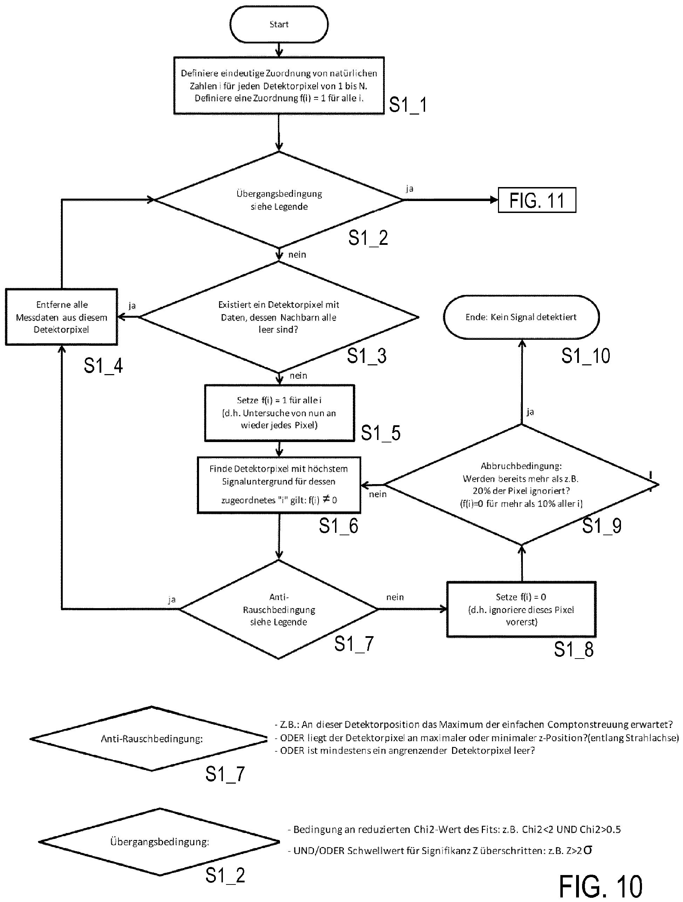

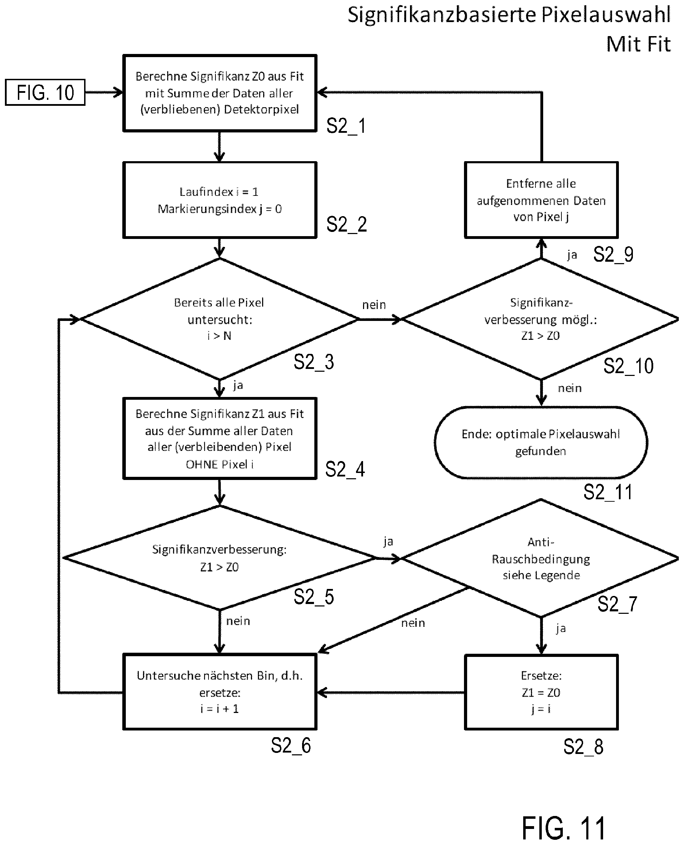

Die erfindungsgemäße Röntgenfluoreszenz-Messung wird an einem Objekt 1 durchgeführt, in das vorab eine Lösung, welche die Targetpartikel enthält, injiziert wurde. Das Objekt 1 wird in die Röntgenfluoreszenz-Messvorrichtung 100 eingeführt, so dass der Röntgenstrahl 2 auf ein interessierendes Teil des Objekts 1 gerichtet werden kann. Die Quelleneinrichtung 10 wird betätigt und der Röntgenstrahl 2 wird mit der Scaneinrichtung 20 über das Objekt 1 gescannt. Mit der Detektorarrayeinrichtung 30 wird die Röntgenstrahlung erfasst, die aus dem Objekt in eine Vielzahl von Raumrichtungen abgestrahlt wird. Detektorsignale werden mit der Steuereinrichtung 40 aufgenommen und verarbeitet, wie im Folgenden erläutert wird und in den Flussdiagrammen der Figuren 10 und 11 gezeigt ist.

Die erfindungsgemäße Röntgenfluoreszenz-Messung basiert insbesondere auf den folgenden Überlegungen der Erfinder. Neben der Kenntnis über die Richtungsabhängigkeit, bzw. Anisotropie des Untergrunds werden durch das Verfahren zwei wesentliche Forderungen erfüllt:
(1) Das Verfahren soll kein a priori Wissen über die Position der Targetpartikel entlang des scannenden Röntgenstrahls 2 voraussetzen. Herkömmliche RFB-Verfahren setzen das aber voraus, wodurch dann mehrfach gescannt werden muss (eben weil man ja beim Patienten gerade nicht weiß, wo der Tumor sitzt) und damit die Dosis deutlich höher ist; und
(2) Die Mehrfach-Compton-Streuung soll maximal reduziert werden. Einfach Comptongestreute Photonen können nicht abgeblockt werden, weil sie aus demselben Gebiet wie die Fluoreszenz-Photonen kommen.

Forderung (1) schließt Kollimatoren und/oder Messmethoden (wie z.B. in DE 10 2012 023 344 A1) aus, die nur ein eingeschränktes Sichtvolumen entlang des scannenden Strahls zulassen. Viele andere Verfahren nutzen dennoch diese Einschränkung, da diese den Untergrund stark reduzieren kann - jedoch ebenso das Signal zu stark abschwächt, so dass die Sensitivität nicht maximal werden kann. Forderung (2) erlaubt aber solche Kollimatoren, die zwar nicht das Nadelstrahlvolumen beschneiden, aber sämtliche Gebiete außerhalb des Strahlvolumens maximal abblocken.

Beide Forderungen können erfüllt werden, indem die radiale Blendenlamellen 32 entlang des scannenden Röntgenstrahls 2 angeordnet sind. Diese schränken nicht die Sicht auf das ganze Strahlvolumen ein, blocken aber Photonen, die außerhalb des Strahlvolumens noch einmal gestreut wurden.

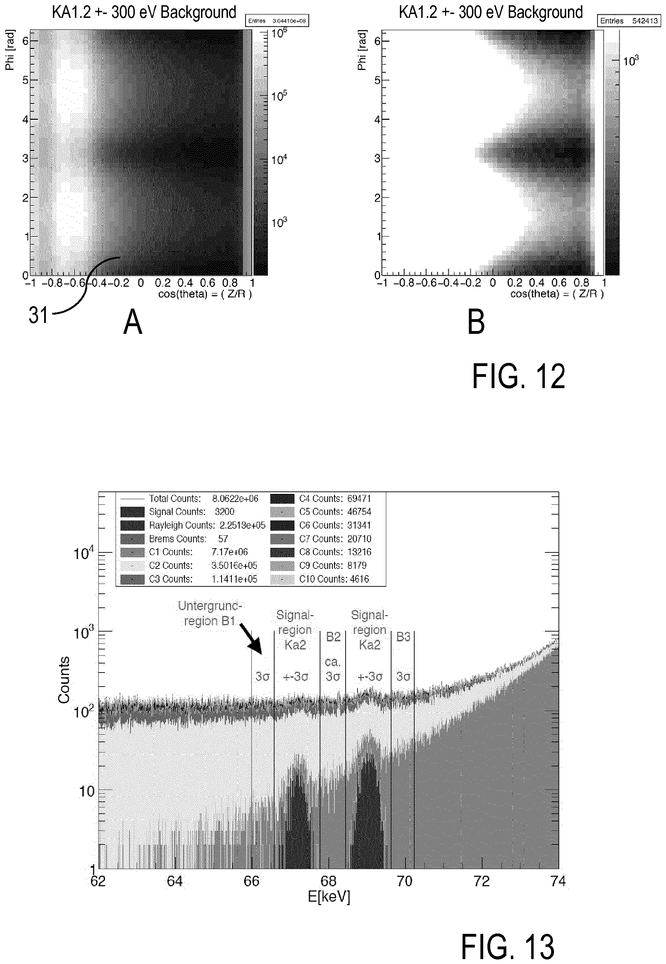

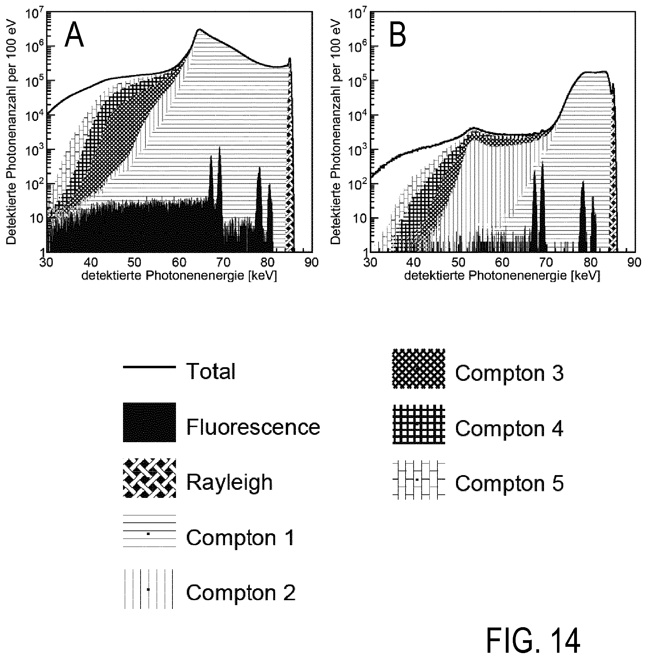

Der wesentliche Gewinn bei der Untergrund-Reduktion, z. B. um den Faktor 570, wird durch die oben beschriebene so genannte "räumliche Filterung" der Detektorsignale, d.h. durch die Identifizierung einer Teilmenge von allen gegebenen Detektorelementen, erzielt, nachdem bereits eine erste Untergrund-Reduktion durch die Blendenlamellen 32 gegeben ist. Dieses Verfahren beruht auf der in Figur 12 verdeutlichten Anisotropie des Untergrunds (siehe auch Figur 14). Figur 12 zeigt schematisch eine Abwicklung der Detektorelemente 31 und die bei der Signalverarbeitung berücksichtigten Detektorelemente 31 bei einem herkömmlichen Verfahren (Figur 12A) und beispielhaft beim erfindungsgemäßen Verfahren (Figur 12B). Die Identifizierung der ausgewählten Detektorelemente 31 basiert auf den folgenden Überlegungen.
(1) Jedes Detektorelement (oder Pixel) 31 (z. B. auf einer Zylinderfläche entlang der Strahlrichtung um das Objekt 1 herum) ist ein eigener Detektor mit einer bestimmten Energieauflösung, d.h. jedes Pixel misst ein Energiespektrum.
(2) Jedes der Detektorelemente 31 hat Einträge von sowohl Signal- als auch Untergrund-photonen. Die Signalphotonen können hierbei von allen Orten innerhalb des Strahlvolumens emittiert und detektiert werden. Damit RFB auch bei Patienten funktionieren kann, also eine weiterhin hohe Sensitivität auch in großen Objekten ermöglicht wird, muss der Untergrund weitaus stärker als in bisherigen Verfahren reduziert werden, ohne die Anzahl der Signalphotonen übermäßig zu verringern.
(3) Die erfindungsgemäße Untergrund-Reduktion basiert auf einer Pixelauswahl, so dass nicht mehr alle Pixel ausgelesen werden, sondern nur bestimmte. Liest man zu viele (oder gar alle) Pixel aus, wird so viel Untergrund detektiert, dass die Signal-Photonen vollständig überdeckt werden. Liest man hingegen nur sehr wenige Pixel aus, mag zwar der Untergrund reduziert sein, das Signal aber entsprechend auch. Das erfindungsgemäße Verfahren führt in die Nähe von oder bis in ein eindeutiges Optimum, indem es iterativ jeweils genau das Pixel aus der Signalverarbeitung verwirft, dessen Entfernung die Signifikanz des Summen-Signals der verbleibenden Pixel steigert. Das Verfahren der Pixelauswahl bricht dann ab, wenn das nächste zu entfernende Pixel keine Signifikanzsteigerung mehr bringt. Dann ist die Situation in Figur 12B erreicht: das Summen-Spektrum der noch vorhandenen Pixel hat die maximale Signal-Signifikanz, wodurch man einen Signalverlauf erreicht, wie in Figur 13 beispielhaft gezeigt ist, das Fluoreszenz-Signal also jetzt erkennbar ist.

Die Figuren 10 und 11 zeigen die zweistufige Variante der Identifizierung der signifikanten Detektorelemente, wobei in einem ersten Auswahlschritt (Figur 10, untergrundbasierte Pixelauswahl ohne Fit) Detektorelemente verworfen werden, die überwiegend Untergrund-Röntgenstreustrahlung detektieren, und in einem zweiten Auswahlschritt (Figur 11, signifikanzbasierte Pixelauswahl mit Fit) weitere Detektorelemente verworfen werden, deren Detektorsignale keine Erhöhung der statistischen Signifikanz eines Summensignals der verbleibenden Detektorelemente liefern. Alternativ kann sich die Ausführung der Identifizierung der signifikanten Detektorelemente auf das Verfahren gemäß Figur 10 beschränken.

Vor der Identifizierung der signifikanten Detektorelemente tastet der Nadel-Röntgenstrahl 2 das Objekt 1 entlang des transversalen Querschnitts ab, wobei jeder Schritt Scanposition heißt. Es werden also alle Positionen in der Projektionsebene des Objekts 1 abgefahren. Der Röntgenstrahl 2 trifft senkrecht auf die Projektionsebene.

Anschließend werden zu jeder Scanposition individuell die identifizierten Detektorelemente ausgewählt (das kann parallel zu den Detektionen erfolgen oder anschließend, Daten werden während der Detektionen also von allen Detektorelementen gespeichert):
Wenn ein Fluoreszenz-Signal vorhanden ist, also die zwei Gold-Fluoreszenz-Linien (siehe Figur 13), dann sind diese im Energiespektrum des Summen-Signals der betrachteten Detektorelemente durch einen reinen Untergrundbereich voneinander getrennt und links und rechts an die Signalregionen gibt es ebenfalls Untergrundbereiche (siehe Figur 13): in dem Untergrund-Bereich kann es nur Untergrund-Photonen geben und keine Signal-Photonen. Diese Bereiche sind also ein Referenzwert. Wenn es kein Fluoreszenz-Signal gibt, würden sich die Signalbereiche statistisch nicht signifikant von den Untergrundbereichen unterscheiden.

Das Verfahren gemäß Figur 10 verwirft mit den Schritten S1_3 bis S1_7 solange Detektorelemente von der weiteren Betrachtung (ignoriert also die detektierten Energiespektren dieser Pixel), bis die Einträge in den drei Untergrundbereichen B1, B2 und B3 (Figur 13) so stark reduziert wurden, dass - wenn vorhanden - die statistische Signifikanz der Untergrund-Überhöhung in den beiden Signal-Bereichen einen Wert von z. B. ≥ 2 überschreitet (Test bei Schritt S1_2). Sollte kein Signal vorhanden sein, würde dieses Abbruchkriterium nie erreicht werden und der Algorithmus stoppt beim letzten verbleibenden Pixel (bzw. wenn z. B. 80% aller Pixel bereits verworfen sind) (Schritt S1_10).

Wenn der Test bei Schritt S1_2 (Übergangsbedingung) erfüllt ist, wird nun der zweite Algorithmus gestartet (Figur 11): hier werden jetzt für die weitere Pixel-Auswahl alle Spektren der noch verbleibenden Pixel in ein Summen-Spektrum aufaddiert und die Signifikanz des Signals direkt durch Fit-Funktionen über die beiden Signalbereiche bestimmt (Schritte S2_1 bis S2_8). Der entscheidende Vorteil dieses Algorithmus ist, dass Fit-Funktionen die Untergrundbereiche besser abbilden (im ersten Schritt von Figur 10 wird der Untergrund über alle Bereiche als konstant angenommen, was aber nur eine Näherung ist) und durch die Fits das Fluoreszenz-Signal vom Untergrund unterschieden werden kann, d.h. erst durch die Fits kann aus dem Summen-Spektrum die Anzahl an Fluoreszenz-Photonen vs Untergrund-Photonen bestimmt werden - und damit die Pixel verworfen werden, die mehr zum Untergrund als zum Signal beitragen.

Diese "Fit-basierte" Pixelauswahl stoppt, sobald das nächste zu entfernende Detektorelement die Signifikanz nicht weiter erhöht (Schritt S2_10), sondern reduziert (einfach weil man zwar weiteren Untergrund entfernen würde, ab da aber auch zu viel Signal, man also nichts mehr gewinnen kann). Im Ergebnis wird bei Schritt S2_11 die optimale Auswahl der identifizierten Detektorelemente ausgegeben. Dann wird die Scan-Position, für welche die die Targetpartikel erfasst wurden, als Target-Scanposition ausgegeben.

Anschließend wird der Verbund aus Quellen-, Detektorarray- und Scaneinrichtungen 10, 20, 30 mit der Schwenkeinrichtung 60 verschwenkt, um eine Abtastlinie entsprechend der Target-Scanposition in einer zweiten Projektionsebene (x-z-Ebene) abzutasten. Die Scanposition entlang der Abtastlinie ergibt die Lage der Targetpartikel in z-Richtung und damit gemeinsam mit der Scanposition in der ersten Projektionsebene die Koordinaten der Targetpartikel.

Figur 14 zeigt eine weitere Demonstration der Vorteile der Erfindung anhand der simulierten Röntgenspektren der Detektorelemente 31, die in Figur 12 gezeigt sind. Figur 14A zeigt das Röntgenspektrum, das gemessen werden würde, wenn zwar der Kollimator benutzt, aber das erfindungsgemäße Verfahren nicht angewendet wird (Verwendung von allen Detektorelementen gemäß Figur 12A). Das Messsignal ist stark von der Compton-Streuung (1- bis 5fach) überlagert.

Figur 14B zeigt das Röntgenspektrum, das erfindungsgemäß gemessen werden würde (Verwendung von identifizierten Detektorelementen gemäß Figur 12B) und sich durch eine erhebliche Untergrund-Reduktion auszeichnet. Die statistische Signifikanz des Signals aus beiden Fluoreszenzlinien beträgt in Figur 14B mehr als das 10-fache der Standardabweichung.

## Patentansprüche

1. Verfahren zur Röntgenfluoreszenz-Messung, wobei das Vorhandensein von fluoreszierenden Targetpartikeln in einem zu untersuchenden Objekt (1) erfasst und vorhandene Targetpartikel im Objekt (1) lokalisiert werden, umfassend die Schritte
(a) Erzeugung eines Röntgenstrahls (2) mit einer Quelleneinrichtung (10), wobei der Röntgenstrahl (2) durch das Objekt (1) in einer Röntgenstrahlrichtung parallel zu einer ersten Projektionsrichtung verläuft,
(b) Bestrahlung des Objekts (1) mit dem Röntgenstrahl (2) an einer Vielzahl von Scanpositionen in einer ersten Projektionsebene, wobei die Scanpositionen mit einer Scaneinrichtung (20) eingestellt werden, mit der die Quelleneinrichtung und das Objekt (1) relativ zueinander bewegt werden,
(c) Detektion von Röntgenstrahlung, die aus dem Objekt (1) in eine Vielzahl von Raumrichtungen abgestrahlt wird, an jeder Scanposition mit einer Detektorarrayeinrichtung (30), die mit der Quelleneinrichtung (10) fest verbunden ist, **dadurch gekennzeichnet dass** die Detektorarrayeinrichtung (30) eine Vielzahl von spektral selektiven Detektorelementen (31), die zur Detektion der Röntgenstrahlung in der Vielzahl von Raumrichtungen angeordnet sind, und eine Vielzahl von Blendenlamellen (32) aufweist, die sich in Radialrichtungen relativ zu der Röntgenstrahlrichtung erstrecken, die Detektorelemente (31) von im Objekt (1) gestreuter Röntgenstrahlung abschirmen und so angeordnet sind, dass die Detektorelemente (31) Röntgenstrahlung von jeglichen Orten innerhalb des Strahlvolumens des Röntgenstrahls im Objekt (1) detektieren können, und
(d) Verarbeitung von Detektorsignalen der Detektorelemente, um in der detektierten Röntgenstrahlung Röntgenfluoreszenz von Targetpartikeln zu erfassen und bei Erfassung der Röntgenfluoreszenz die Targetpartikel im Objekt (1) zu lokalisieren, wobei
- für jede von einer Vielzahl von vorbestimmten Scanpositionen mit jedem Detektorelement ein Energiespektrum der aus dem Objekt austretenden Röntgenstrahlung gemessen und eine Teilmenge von signifikanten Detektorelementen (31) gesucht wird, deren Summensignal die Erfassung der Röntgenfluoreszenz der Targetpartikel mit einer im Vergleich zu den übrigen Detektorelementen (31) erhöhten statistischen Signifikanz ermöglicht, und
- wenn an mindestens einer Scanposition signifikante Detektorelemente (31) gefunden werden, das Vorhandensein von Targetpartikeln erfasst und diese Scanposition als Target-Scanposition ermittelt wird, an der die Targetpartikel in der ersten Projektionsebene lokalisiert sind, oder, wenn an keiner Scanposition signifikante Detektorelemente (31) gefunden werden, erfasst wird, dass keine Targetpartikel vorhanden sind.

2. Verfahren gemäß Anspruch 1, bei dem
- für jede der Vielzahl von vorbestimmten Scanpositionen die Teilmenge der signifikanten Detektorelemente (31) so gesucht wird, dass das Summensignal der signifikanten Detektorelemente (31) die Erfassung der Röntgenfluoreszenz der Targetpartikel mit einer maximalen statistischen Signifikanz ermöglicht, und/oder
- für jede der Vielzahl von vorbestimmten Scanpositionen die Teilmenge von signifikanten Detektorelementen (31) gesucht wird, indem Detektorelemente (31) verworfen werden, deren Detektorsignale keine Erhöhung der statistischen Signifikanz des Summensignals der verbleibenden Detektorelemente (31) liefern.

3. Verfahren gemäß einem der vorhergehenden Ansprüche, bei dem
- für jede der Vielzahl von vorbestimmten Scanpositionen die Teilmenge von signifikanten Detektorelementen (31) mit einer zweistufigen Auswahl gesucht wird, wobei
- in einem ersten Auswahlschritt Detektorelemente (31) verworfen werden, die überwiegend Untergrund-Röntgenstreustrahlung detektieren, und
- in einem zweiten Auswahlschritt weitere Detektorelemente (31) verworfen werden, deren Detektorsignale keine Erhöhung der statistischen Signifikanz des Summensignals der verbleibenden Detektorelemente (31) liefern.

4. Verfahren gemäß einem der vorhergehenden Ansprüche, bei dem
- für jede der Vielzahl von vorbestimmten Scanpositionen die Teilmenge von signifikanten Detektorelementen (31) in einer vorgewählten Teilmenge von Detektorelementen (31) gesucht wird, die vorab aufgrund von a priori Information über das untersuchte Objekt (1) ermittelt wird.

5. Verfahren gemäß einem der vorhergehenden Ansprüche, bei dem
- die Schritte (a) bis (d) bei einer Vorbereitungsmessung mit einem ersten Röntgenstrahl mit einem ersten Durchmesser ausgeführt werden, um bei Erfassung des Vorhandenseins der Targetpartikel eine Vorbereitungs-Target-Scanposition zu ermitteln, die einen Target-Scanbereich in der ersten Projektionsebene darstellt, und anschließend
- die Schritte (a) bis (d) bei einer Hauptmessung mit einem zweiten Röntgenstrahl mit einem zweiten Durchmesser ausgeführt werden, der gleich dem ersten Durchmesser oder geringer als der erste Durchmesser ist, wobei die gesuchte Target-Scanposition innerhalb des Target-Scanbereichs ermittelt wird.

6. Verfahren gemäß einem der vorhergehenden Ansprüche, bei dem
- die Detektorarrayeinrichtung (30) eine Anordnung der Detektorelemente (31) auf einer Fläche umfasst, die einen Halbraum in Vorwärtsrichtung des Röntgenstrahls abdeckt, und/oder
- die Detektorarrayeinrichtung (30) eine Anordnung der Detektorelemente (31) auf einer Kugelfläche oder einer Zylinderfläche umfasst.

7. Verfahren gemäß einem der vorhergehenden Ansprüche, wobei, wenn mindestens eine Target-Scanposition ermittelt wird, die folgenden weiteren Schritte vorgesehen sind:
- Verschwenkung der Quellen- und Detektorarrayeinrichtungen (10, 30) derart, dass der Röntgenstrahl (2) parallel zu einer zweiten Projektionsrichtung verläuft, die von der ersten Projektionsrichtung abweicht,
- Bestrahlung des Objekts (1) mit dem Röntgenstrahl (2) an einer Vielzahl von Scanpositionen entlang einer Abtastlinie in einer zweiten Projektionsebene, die von der ersten Projektionsebene abweicht, wobei die Abtastlinie die Target-Scanposition enthält, und
- Erfassung der Position der Targetpartikel entlang der Abtastlinie.

8. Verfahren gemäß Anspruch 7, bei dem
- die Verschwenkung der Quellen- und Detektorarrayeinrichtungen (10, 30) derart erfolgt, dass die zweite Projektionsrichtung senkrecht zu der ersten Projektionsrichtung und die zweite Projektionsebene senkrecht zu der ersten Projektionsebene ausgerichtet sind.

9. Verfahren gemäß einem der vorhergehenden Ansprüche, bei dem
- mindestens ein Absorptionsprojektionsbild des Objekts (1) aufgenommen wird,
- das Objekt (1) ein menschlicher Proband ist, und/oder
- die Targetpartikel Atome mit einer Massenzahl im Bereich der Massenzahlen von lod bis Gold enthalten und mit einer Markersubstanz oder Medikamenten funktionalisiert sind.

10. Röntgenfluoreszenz-Messvorrichtung (100), die zur Lokalisierung von fluoreszierenden Targetpartikeln in einem zu untersuchenden Objekt (1) eingerichtet ist, umfassend
- eine Halteeinrichtung (50), die zur Aufnahme des Objekts (1) eingerichtet ist,
- eine Quelleneinrichtung (10), die zur Erzeugung eines Röntgenstrahls eingerichtet ist, dessen Strahlvolumen im zu untersuchenden Objekt (1) in einer Röntgenstrahlrichtung parallel zu einer ersten Projektionsrichtung verläuft,
- eine Detektorarrayeinrichtung (30), die mit der Quelleneinrichtung (10) fest verbunden ist, zur Detektion von Röntgenstrahlung eingerichtet ist, die aus dem Objekt (1) in eine Vielzahl von Raumrichtungen abgestrahlt wird, wobei die Röntgenfluoreszenz-Messvorrichtung (100) **dadurch gekennzeichnet ist, dass** die Detektorarrayeinrichtung (30) eine Vielzahl von spektral selektiven Detektorelementen (31), die zur Detektion der Röntgenstrahlung in der Vielzahl von Raumrichtungen angeordnet sind, und eine Vielzahl von Blendenlamellen (32) aufweist, die sich in Radialrichtungen relativ zu der Röntgenstrahlrichtung erstrecken, die Detektorelemente (31) von im Objekt (1) gestreuter Röntgenstrahlung abschirmen und so angeordnet sind, dass die Detektorelemente (31) Röntgenstrahlung von jeglichen Orten innerhalb des Strahlvolumens im Objekt (1) detektieren können,
- eine Scaneinrichtung (20), mit der die Quellen- und Detektorarrayeinrichtungen (10, 30) und die Halteeinrichtung (50) relativ zueinander derart beweglich sind, dass der Röntgenstrahl (2) das Objekt (1) in einer ersten Projektionsebene an einer Vielzahl von Scanpositionen abtasten kann, und
- eine Steuereinrichtung (40), die zur Verarbeitung von Detektorsignalen der Detektorelemente eingerichtet ist, um in der detektierten Röntgenstrahlung Röntgenfluoreszenz von Targetpartikeln zu erfassen und bei Erfassung der Röntgenfluoreszenz die Targetpartikel im Objekt (1) zu lokalisieren, wobei
- die Steuereinrichtung (40) dazu eingerichtet ist, für jede von einer Vielzahl von vorbestimmten Scanpositionen eine Teilmenge von signifikanten Detektorelementen (31) zu suchen, deren Summensignal die Erfassung der Röntgenfluoreszenz der Targetpartikel mit einer im Vergleich zu den übrigen Detektorelementen (31) erhöhten statistischen Signifikanz ermöglicht, und, wenn an mindestens einer Scanposition signifikante Detektorelemente (31) gefunden werden, das Vorhandensein von Targetpartikeln zu erfassen und diese Scanposition als Target-Scanposition zu ermitteln, an der die Targetpartikel in der ersten Projektionsebene lokalisiert sind, oder, wenn an keiner Scanposition signifikante Detektorelemente (31) gefunden werden, zu erfassen, dass keine Targetpartikel vorhanden sind.

11. Röntgenfluoreszenz-Messvorrichtung gemäß Anspruch 10, bei der
- die Steuereinrichtung (40) zur Suche nach der Teilmenge von signifikanten Detektorelementen (31) eingerichtet ist, deren Summensignal die Erfassung der Röntgenfluoreszenz der Targetpartikel mit einer maximalen statistischen Signifikanz ermöglicht, und/oder
- die Steuereinrichtung (40) zur Suche der Teilmenge von signifikanten Detektorelementen (31) eingerichtet ist, indem Detektorelemente (31) verworfen werden, deren Detektorsignale keine Erhöhung der statistischen Signifikanz des Summensignals der verbleibenden Detektorelemente (31) liefern, und/oder
- die Detektorarrayeinrichtung (30) und die Steuereinrichtung (40) zur Aufnahme mindestens eines Absorptionsprojektionsbildes des Objekts (1) eingerichtet sind.

12. Röntgenfluoreszenz-Messvorrichtung gemäß einem der Ansprüche 10 bis 11, bei der
- die Steuereinrichtung (40) zur Suche der Teilmenge von signifikanten Detektorelementen (31) basierend auf einer zweistufigen Auswahl eingerichtet ist, wobei
- in einem ersten Auswahlschritt Detektorelemente (31) verworfen werden, die überwiegend Untergrund-Röntgenstreustrahlung detektieren, und
- in einem zweiten Auswahlschritt weitere Detektorelemente (31) verworfen werden, deren Detektorsignale keine Erhöhung der statistischen Signifikanz des Summensignals der verbleibenden Detektorelemente (31) liefern.

13. Röntgenfluoreszenz-Messvorrichtung gemäß einem der Ansprüche 10 bis 12, bei der
- die Detektorarrayeinrichtung (30) eine Anordnung der Detektorelemente (31) auf einer Fläche umfasst, die einen Halbraum in Vorwärtsrichtung des Röntgenstrahls abdeckt, und/oder
- die Detektorarrayeinrichtung (30) eine Anordnung der Detektorelemente (31) auf einer Kugelfläche oder einer Zylinderfläche umfasst.

14. Röntgenfluoreszenz-Messvorrichtung gemäß einem der Ansprüche 10 bis 13, die umfasst
- eine Schwenkeinrichtung (60), mit der die Quellen- und Detektorarrayeinrichtungen (10, 30) so verschwenkbar sind, dass der Röntgenstrahl (2) parallel zu einer zweiten Projektionsrichtung verläuft, die von der ersten Projektionsrichtung abweicht, wobei
- die Scaneinrichtung (20) für eine Bewegung der Quellen- und Detektorarrayeinrichtungen (10, 30) und der Halteeinrichtung (50) relativ zueinander derart eingerichtet ist, dass der Röntgenstrahl (2) das Objekt (1) entlang einer Abtastlinie in einer zweiten Projektionsebene, die von der ersten Projektionsebene abweicht, abtasten kann, und
- die Steuereinrichtung (40), wenn mindestens eine Target-Scanposition ermittelt worden ist, zur Erfassung der Position der Targetpartikel entlang der Abtastlinie eingerichtet ist.

15. Röntgenfluoreszenz-Messvorrichtung gemäß Anspruch 14, bei der
- die Schwenkeinrichtung (60) zur Verschwenkung der Quellen- und Detektorarrayeinrichtungen (10, 30) derart eingerichtet ist, dass die zweite Projektionsrichtung senkrecht zu der ersten Projektionsrichtung und die zweite Projektionsebene senkrecht zu der ersten Projektionsebene ausgerichtet sind.

## Claims

1. Method for an X-ray fluorescence measurement, wherein the presence of fluorescing target particles is detected in an object (1) to be examined and target particles that are present are localized in the object (1), comprising the steps of
(a) generating an X-ray beam (2) by means of a source device (10), wherein the X-ray beam (2) extends through the object (1) in an X-ray beam direction parallel to a first projection direction,
(b) irradiating the object (1) with the X-ray beam (2) at a multiplicity of scan positions in a first projection plane, wherein the scan positions are set by a scanning device (20), by means of which the source device and the object (1) are moved relative to one another,
(c) detecting X-ray radiation, emitted from the object (1) in a plurality of spatial directions, at each scan position using a detector array device (30), which is securely connected to the source device (10), **characterized in that**
the detector array device (30) comprises a multiplicity of spectrally selective detector elements (31), which are arranged to detect the X-ray radiation in the multiplicity of spatial directions, and a plurality of screen lamellas (32), which extend in radial directions relative to the X-ray beam direction, which shield the detector elements (31) from X-ray radiation scattered in the object (1) and which are arranged in such a way that the detector elements (31) are able to detect X-ray radiation from all locations within the beam volume of the X-ray beam in the object (1), and
(d) processing detector signals of the detector elements in order to detect X-ray fluorescence of target particles in the detected X-ray radiation and in order to localize the target particles in the object (1) if the X-ray fluorescence is detected, wherein
- for each of a multiplicity of predetermined scan positions an energy spectrum of the X-ray radiation emerging from the object is measured with each detector element and a subset of significant detector elements (31) is sought after, the sum signal of said detector elements allowing the detection of the X-ray fluorescence of the target particles with a statistical significance that is increased in comparison with the remaining detector elements (31), and
- if significant detector elements (31) are found at at least one scan position, the presence of target particles is detected and this scan position is established as a target scan position, at which the target particles are localized in the first projection plane, or, if significant detector elements (31) are found at no scan position, the presence of no target particles is detected.

2. Method according to claim 1, in which
- for each of the multiplicity of predetermined scan positions the subset of the significant detector elements (31) is sought after in such a way that the sum signal of the significant detector elements (31) facilitates the detection of the X-ray fluorescence of the target particles with a maximum statistical significance, and/or
- for each of the multiplicity of predetermined scan positions the subset of significant detector elements (31) is sought after by discarding detector elements (31), the detector signals of which do not deliver any elevation of the statistical significance of the sum signal of the remaining detector elements (31).

3. Method according to one of the preceding claims, in which
- for each of the multiplicity of predetermined scan positions the subset of significant detector elements (31) is sought after with a two-stage selection, wherein
- in a first selection step detector elements (31) are discarded, which detect predominantly background X-ray scatter radiation, and
- in a second selection step further detector elements (31) are discarded, the detector signals of which do not deliver any increase of the statistical significance of the sum signal of the remaining detector elements (31).

4. Method according to one of the preceding claims, in which
- for each of the multiplicity of predetermined scan positions the subset of significant detector elements (31) is sought after in a preselected subset of detector elements (31), which is established beforehand on the basis of a priori information about the object (1) being examined.

5. Method according to one of the preceding claims, in which
- the steps (a) to (d) are performed in a preparatory measurement with a first X-ray beam with a first diameter, in order to establish a preparatory target scan position representing a target scan region in the first projection plane, if the presence of the target particles is detected, and thereafter
- the steps (a) to (d) are performed in a main measurement with a second X-ray beam with a second diameter, which is the same as the first diameter or smaller than the first diameter, whereby the sought-after target scan position is established inside the target scan region.

6. Method according to one of the preceding claims, in which
- the detector array device (30) comprises an arrangement of the detector elements (31) on a surface that covers a half-space in forward direction of the X-ray beam, and/or
- the detector array device (30) comprises an arrangement of the detector elements (31) on a spherical surface or a cylindrical surface.

7. Method according to one of the preceding claims, whereby, if at least one target scan position is established, the following further steps are provided:
- swivelling of the source and detector array devices (10, 30) in such a way that the X-ray beam (2) extends parallel to a second projection direction, which deviates from the first projection direction,
- irradiating of the object (1) with the X-ray beam (2) at a multiplicity of scan positions along a scanning line in a second projection plane, which deviates from the first projection plane, whereby the scanning line contains the target scan position, and
- detecting of the position of the target particles along the scanning line.

8. Method according to claim 7, in which
- the swivelling of the source and detector array devices (10, 30) takes place in such a way that the second projection direction is orientated perpendicular to the first projection direction and the second projection plane is orientated perpendicular to the first projection plane.

9. Method according to one of the preceding claims, in which
- at least one absorption projection image of the object (1) is collected,
- the object (1) is a human test subject, and/or
- the target particles contain atoms with a mass number in the range of the mass numbers of iodine to gold and are functionalized with a marker substance or medications.

10. X-ray fluorescence measuring apparatus (100), which is configured to localize fluorescing target particles in an object (1) to be examined, comprising
- a holding device (50), which is configured to accommodate the object (1),
- a source device (10), which is configured to generate an X-ray beam, the beam volume of which extends in the object (1) to be examined in an X-ray beam direction parallel to a first projection direction,
- a detector array device (30), which is securely connected to the source device (10), is configured to detect X-ray radiation, emitted from the object (1) in a plurality of spatial directions, wherein the X-ray fluorescence measuring apparatus (100) is **characterized in that** the detector array device (30) comprises a multiplicity of spectrally selective detector elements (31), which are arranged to detect the X-ray radiation in the multiplicity of spatial directions, and a plurality of screen lamellas (32), which extend in radial directions relative to the X-ray beam direction, which shield the detector elements (31) from X-ray radiation scattered in the object (1) and which are arranged in such a way that the detector elements (31) are able to detect X-ray radiation from all locations within the beam volume in the object (1),
- a scanning device (20), by means of which the source and detector array devices (10, 30) and the holding device (50) can be moved relative to one another in such a way that the X-ray beam (2) can scan the object (1) in a first projection plane at a multiplicity of scan positions, and
- a control device (40), which is configured to process detector signals of the detector elements, in order to detect X-ray fluorescence of target particles in the detected X-ray radiation and in order to localize the target particles in the object (1) if the X-ray fluorescence is detected, wherein
- the control device (40) is configured to seek after a subset of significant detector elements (31) for each of a multiplicity of predetermined scan positions, the sum signal of said detector elements allowing the detection of the X-ray fluorescence of the target particles with a statistical significance that is increased in comparison with the remaining detector elements (31), and, if significant detector elements (31) are found at at least one scan position, to detect the presence of target particles and to establish this scan position as target scan position, at which the target particles are localized in the first projection plane, or, if significant detector elements (31) are found at no scan position, to detection that no target particles are present.

11. X-ray fluorescence measuring apparatus according to claim 10, in which
- the control device (40) is configured to seek after the subset of significant detector elements (31), the sum signal of said detector elements facilitating the detection of the X-ray fluorescence of the target particles with a maximum statistical significance, and/or
- the control device (40) is configured to seek after the subset of significant detector elements (31) by discarding detector elements (31), the detector signals of which do not deliver any increase of the statistical significance of the sum signal of the remaining detector elements (31), and/or
- the detector array device (30) and the control device (40) are configured to take at least one absorption projection image of the object (1).

12. X-ray fluorescence measuring apparatus according to one of claims 10 to 11, in which
- the control device (40) is configured to seek after the subset of significant detector elements (31) on the basis of a two-stage selection, wherein
- in a first selection step detector elements (31) are discarded, which detect predominantly background X-ray scatter radiation, and
- in a second selection step further detector elements (31) are discarded, the detector signals of which do not deliver any elevation of the statistical significance of the sum signal of the remaining detector elements (31).

13. X-ray fluorescence measuring apparatus according to one of claims 10 to 12, in which
- the detector array device (30) comprises an arrangement of the detector elements (31) on a surface that covers a half-space in forward direction of the X-ray beam, and/or
- the detector array device (30) comprises an arrangement of the detector elements (31) on a spherical surface or a cylindrical surface.

14. X-ray fluorescence measuring apparatus according to one of claims 10 to 13, comprising
- a swivel device (60), with which the source and detector array devices (10, 30) are swivellable, such that the X-ray beam (2) extends parallel to a second projection direction, which deviates from the first projection direction, wherein
- the scanning device (20) is configured for a movement of the source and detector array devices (10, 30) and of the holding device (50) relative to one another in such a way that the X-ray beam (2) can scan the object (1) along a scanning line in a second projection plane, which deviates from the first projection plane, and
- the control device (40) is configured to detect the position of the target particles along the scanning line, if at least one target scan position has been established.

15. X-ray fluorescence measuring apparatus according to claim 14, in which
- the swivel device (60) for swivelling of the source and detector array devices (10, 30) is configured in such a way that the second projection direction is orientated perpendicular to the first projection direction and the second projection plane is orientated perpendicular to the first projection plane.

## Revendications

1. Procédé de mesure de la fluorescence de rayons X, dans lequel la présence de particules cibles fluorescentes dans un objet (1) à examiner est détectée et des particules cibles présentes dans l'objet (1) sont localisées, comprenant les étapes
(a) de génération d'un faisceau de rayons X (2) avec un système de source (10), dans lequel le faisceau de rayons X (2) s'étend à travers l'objet (1) dans une direction de rayons X de manière parallèle à une première direction de projection,
(b) d'exposition de l'objet (1) au faisceau de rayons X (2) sur une pluralité de positions de balayage dans un premier plan de projection, dans lequel les positions de balayage sont réglées avec un système de balayage (20), avec lequel le système de source et l'objet (1) sont déplacés l'un par rapport à l'autre,
(c) de détection d'un rayonnement X, qui est émis depuis l'objet (1) dans une pluralité de directions spatiales, sur chaque position de balayage avec un système de réseau de détecteurs (30), qui est relié de manière fixe au système de source (10), **caractérisé en ce que**
le système de réseau de détecteurs (30) présente une pluralité d'éléments détecteurs (31) à sélectivité spectrale, qui sont disposés pour la détection du rayonnement X dans la pluralité de directions spatiales, et une pluralité de lamelles de diaphragme (32), qui s'étendent dans des directions radiales par rapport à la direction de rayons X, protègent les éléments détecteurs (31) d'un rayonnement X diffusé dans l'objet (1) et sont disposés de telle sorte que les éléments détecteurs (31) peuvent détecter un rayonnement X depuis des quelconques emplacements à l'intérieur du volume de faisceau du faisceau de rayons X dans l'objet (1), et
(d) de traitement de signaux de détecteur des éléments détecteurs pour détecter, dans le rayonnement X détecté, la fluorescence de rayons X de particules cibles et pour localiser les particules cibles dans l'objet (1) lors de la détection de la fluorescence de rayons X, dans lequel
- pour chacune d'une pluralité de positions de balayage prédéfinies, un spectre d'énergie du rayonnement X sortant de l'objet est mesuré avec chaque élément détecteur et une quantité partielle d'éléments détecteurs (31) significatifs est recherchée, dont le signal cumulé permet la détection de la fluorescence de rayons X des particules cibles avec une signification statistique élevée en comparaison avec les éléments détecteurs (31) restants, et
- lorsque des éléments détecteurs (31) significatifs sont retrouvés sur au moins une position de balayage, la présence de particules cibles est détectée et cette position de balayage est déterminée en tant que position de balayage cible, sur laquelle les particules cibles sont localisées dans le premier plan de projection, ou,
lorsqu'aucun élément détecteur (31) significatif n'est retrouvé sur une position de balayage, il est détecté qu'aucune particule cible n'est présente.

2. Procédé selon la revendication 1, dans lequel
- pour chacune de la pluralité de positions de balayage prédéfinies, la quantité partielle des éléments détecteurs (31) significatifs est recherchée de telle sorte que le signal cumulé des éléments détecteurs (31) significatifs permet la détection de la fluorescence de rayons X des particules cibles avec une signification statistique maximale, et/ou
- pour chacune de la pluralité de positions de balayage prédéfinies, la quantité partielle d'éléments détecteurs (31) significatifs est recherchée en ce que des éléments détecteurs (31) dont les signaux de détecteur ne fournissent aucune augmentation de la signification statistique du signal cumulé des éléments détecteurs (31) restants, sont rejetés.

3. Procédé selon l'une quelconque des revendications précédentes, dans lequel
- pour chacune de la pluralité de positions de balayage prédéfinies, la quantité partielle d'éléments détecteurs (31) significatifs est recherchée avec une sélection en deux étapes, dans lequel
- dans une première étape de sélection, des éléments détecteurs (31), qui détectent majoritairement un rayonnement de diffusion de rayons X souterrain, sont rejetés, et
- dans une deuxième étape de sélection, d'autres éléments détecteurs (31), dont les signaux de détecteur ne fournissent aucune augmentation de la signification statistique du signal cumulé des éléments détecteurs (31) restants, sont rejetés.

4. Procédé selon l'une quelconque des revendications précédentes, dans lequel
- pour chacune de la pluralité de positions de balayage prédéfinies, la quantité partielle d'éléments détecteurs (31) significatifs est recherchée dans une quantité partielle présélectionnée d'éléments détecteurs (31), qui est déterminée au préalable sur la base d'informations a priori sur l'objet (1) examiné.

5. Procédé selon l'une quelconque des revendications précédentes, dans lequel
- les étapes (a) à (d) sont exécutées, lors d'une mesure de préparation, avec un premier faisceau de rayons X avec un premier diamètre pour déterminer, lors de la détection de la présence des particules cibles, une position de balayage cible de préparation, qui représente une zone de balayage cible dans le premier plan de projection, puis
- les étapes (a) à (d) sont exécutées, lors d'une mesure principale, avec un deuxième faisceau de rayons X avec un deuxième diamètre, qui est égal au premier diamètre ou est inférieur au premier diamètre, dans lequel la position de balayage cible recherchée est déterminée à l'intérieur de la zone de balayage cible.

6. Procédé selon l'une quelconque des revendications précédentes, dans lequel
- le système de réseau de détecteurs (30) comprend un ensemble des éléments détecteurs (31) sur une surface, qui recouvre un demi-espace dans la direction vers l'avant du faisceau de rayons X, et/ou
- le système de réseau de détecteurs (30) comprend un ensemble d'éléments détecteurs (31) sur une surface sphérique ou une surface cylindrique.

7. Procédé selon l'une quelconque des revendications précédentes, dans lequel, lorsqu'au moins une position de balayage cible est déterminée, les autres étapes suivantes sont prévues :
- le pivotement des systèmes de source et de réseau de détecteurs (10, 30) de telle manière que le faisceau de rayons X (2) s'étend de manière parallèle à une deuxième direction de projection qui diverge de la première direction de projection,
- l'exposition de l'objet (1) au faisceau de rayons X (2) sur une pluralité de positions de balayage le long d'une ligne de balayage dans un deuxième plan de projection, qui diverge du premier plan de projection, dans lequel la ligne de balayage contient la position de balayage cible, et
- la détection de la position des particules cibles le long de la ligne de balayage.

8. Procédé selon la revendication 7, dans lequel
- le pivotement des systèmes de source et de réseau de détecteurs (10, 30) est effectué de telle manière que la deuxième direction de projection est orientée de manière perpendiculaire à la première direction de projection et le deuxième plan de projection est orienté de manière perpendiculaire au premier plan de projection.

9. Procédé selon l'une quelconque des revendications précédentes, dans lequel
- au moins une image de projection d'absorption de l'objet (1) est enregistrée,
- l'objet (1) est un sujet humain, et/ou
- les particules cibles contiennent des atomes avec une masse atomique dans la plage des masses atomiques de l'iode à l'or et sont fonctionnalisées avec une substance de marquage ou des médicaments.

10. Dispositif de mesure de fluorescence de rayons X (100), qui est mis au point pour localiser des particules cibles fluorescentes dans un objet (1) à examiner, comprenant
- un système de maintien (50), qui est mis au point pour recevoir l'objet (1),
- un système de source (10), qui est mis au point pour générer un faisceau de rayons X, dont le volume de rayonnement dans l'objet (1) à examiner s'étend dans une direction de faisceau de rayons X de manière parallèle à une première direction de projection,
- un système de réseau de détecteurs (30), qui est relié de manière fixe au système de source (10), pour la détection d'un rayonnement X, qui est émis hors de l'objet (1) dans une pluralité de directions spatiales, dans lequel le dispositif de mesure de fluorescence de rayons X (100) est **caractérisé en ce que** le système de réseau de détecteurs (30) présente une pluralité d'éléments détecteurs (31) à sélectivité spectrale, qui sont disposés dans la pluralité de directions spatiales pour la détection du rayonnement X, et une pluralité de lamelles de diaphragme (32), qui s'étendent dans des directions radiales par rapport à la direction de faisceau de rayons X, qui protègent des éléments détecteurs (31) du rayonnement X diffusé dans l'objet (1) et sont disposés de telle sorte que les éléments détecteurs (31) peuvent détecter un rayonnement X depuis des emplacements quelconques à l'intérieur du volume de rayonnement dans l'objet (1),
- un système de balayage (20), avec lequel les systèmes de source et de réseau de détecteurs (10, 30) et le système de maintien (50) sont mobiles les uns par rapport aux autres de telle manière que le faisceau de rayons X (2) peut balayer l'objet (1) dans un premier plan de projection sur une pluralité de positions de balayage, et
- un système de commande (40), qui est mis au point pour traiter des signaux de détecteur des éléments détecteurs pour détecter, dans le rayonnement X détecté la fluorescence de rayons X de particules de suie et pour localiser, lors de la détection de la fluorescence de rayons X, les particules cibles dans l'objet (1), dans lequel
- le système de commande (40) est mis au point pour rechercher, pour chacune d'une pluralité de positions de balayage prédéfinies, une quantité partielle d'éléments détecteurs (31) significatifs, dont le signal cumulé permet la détection de la fluorescence de rayons X des particules cibles avec une signification statistique augmentée en comparaison avec les éléments détecteurs (31) restants, et, lorsque des éléments détecteurs (31) significatifs sont trouvés sur au moins une position de balayage, pour détecter la présence de particules cibles et pour déterminer cette position de balayage en tant que position de balayable cible, sur laquelle les particules cibles sont localisées dans le premier plan de projection, ou, lorsqu'aucun élément détecteur (31) n'est trouvé sur une position de balayage, pour détecter qu'aucune particule cible n'est présente.

11. Dispositif de mesure de fluorescence de rayons X selon la revendication 10, dans lequel
- le système de commande (40) est mis au point pour rechercher la quantité partielle d'éléments détecteurs (31) significatifs, dont le signal cumulé permet la détection de la fluorescence de rayons X des particules cibles avec une signification statistique maximale, et/ou
- le système de commande (40) est mis au point pour rechercher la quantité partielle d'éléments détecteurs (31) significatifs, en ce que des éléments détecteurs (31), dont les signaux détecteurs ne fournissent aucune augmentation de la signification statistique du signal cumulé des éléments détecteurs (31) restants, sont rejetés, et/ou
- le système de réseau de détecteurs (30) et le système de commande (40) sont mis au point pour enregistrer au moins une image de projection d'absorption de l'objet (1).

12. Dispositif de mesure de fluorescence de rayons X selon l'une quelconque des revendications 10 à 11, dans lequel
- le système de commande (40) est mis au point pour rechercher la quantité partielle d'éléments détecteurs (31) significatifs sur la base d'une sélection en deux phases, dans lequel
- dans une première étape de sélection, des éléments détecteurs (31), qui détectent majoritairement un rayonnement de diffusion de rayons X souterrain, sont rejetés, et
- dans une deuxième étape de sélection, d'autres éléments détecteurs (31), dont les signaux détecteurs ne fournissent aucune augmentation de la signification statistique du signal cumulé des éléments détecteurs (31) restants, sont rejetés.

13. Dispositif de mesure de fluorescence de rayons X selon l'une quelconque des revendications 10 à 12, dans lequel
- le système de réseau de détecteurs (30) comprend un ensemble des éléments détecteurs (31) sur une surface, qui recouvre un demi-espace dans la direction vers l'avant du faisceau de rayons X, et/ou
- le système de réseau de détecteurs (30) comprend un ensemble des éléments détecteurs (31) sur une surface sphérique ou une surface cylindrique.

14. Dispositif de mesure de fluorescence de rayons X selon l'une quelconque des revendications 10 à 13, qui comprend
- un système de pivotement (60), avec lequel les systèmes de source et de réseau de détecteurs (10, 30) peuvent être pivotés de telle sorte que le faisceau de rayons X (2) s'étend de manière parallèle à une deuxième direction de projection, qui diverge de la première direction de projection, dans lequel
- le système de balayage (20) est mis au point pour un déplacement des systèmes de source et de réseau de détecteurs (10, 30) et du système de maintien (50) l'un par rapport à l'autre de telle manière que le faisceau de rayons X (2) peut balayer l'objet (1) le long d'une ligne de balayage dans un deuxième plan de projection, qui diverge du premier plan de projection, et
- le système de commande (40), lorsqu'au moins une position de balayage cible a été déterminée, est mis au point pour détecter la position des particules cibles le long de la ligne de balayage.

15. Dispositif de mesure de fluorescence de rayons X selon la revendication 14, dans lequel
- le système de pivotement (60) est mis au point pour faire pivoter les systèmes de source et de réseau de détecteurs (10, 30) de telle manière que la deuxième direction de projection est orientée de manière perpendiculaire à la première direction de projection et le deuxième plan de projection est orienté de manière perpendiculaire au premier plan de projection.
